# EUROPEAN PATENT APPLICATION

(11) **EP 1 535 891 A1**
(43) Date of publication of application: **01.06.2005**
(21) Application number: 03765309.4
(22) Date of filing: 17.07.2003
(51) Int. Cl.: C07C 35/44, A61K 31/704, A61K 31/56, A61P 9/00, A61P 25/00, A61P 27/02, A61P 35/00, A61P 43/00, A23L 1/30, C07H 15/256, C07J 63/00

(54) **REMEDY**

(30) Priority: 18.07.2002 JP 2002209320
(71) Applicant: TAKARA BIO INC., Otsu-shi, Shiga 520-2193 (JP)
(72) Inventor: OHNOGI, Hiromu, Kusatsu-shi, Shiga 525-0025 (JP); SUGIYAMA, Katsumi, Otsu-shi, Shiga 520-2134 (JP); SAGAWA, Hiroaki, Kusatsu-shi, Shiga 525-0025 (JP); KATO, Ikunoshin, Shiga 529-1851 (JP)
(74) Representative: VOSSIUS & PARTNER
(86) International application number: PCT/JP2003/009060
(87) International publication number: WO 2004/009521

(57) **Abstract**

The present invention relates to a therapeutic agent or prophylactic agent for a disease requiring enhancement of nerve growth factor (NGF) production upon treatment or prevention, an enhancing agent for nerve growth factor production, and a food, beverage or feed for enhancing nerve growth factor production, **characterized in that** each comprises as an effective ingredient a compound selected from the group consisting of soyasaponin compounds, soyasapogenin compounds, glycyrrhizin and salts thereof. In addition, the present invention relates to a novel soyasaponin compound and a novel soyasapogenin compound, having an action for enhancement of NGF production.

## Description

### TECHNICAL FIELD

The present invention mainly relates to a medicament, foodstuff or the like, utilizing the physiological action of a compound derived from an edible plant.

### BACKGROUND ART

Nerve growth factor (NGF) is a protein closely related with life and death of nerve cells, showing actions such as an action for promoting birth of nerve cells, an action for maintaining survival of nerve cells, an action for repairing the nerve cells upon brain damage, and an action of recovering the functions of cranial nerves, thereby preventing aging of the brain. Therefore, there have been expected effects for preventing and treating Alzheimer's dementia, neural disorders in a complication of diabetes, or the like. However, since NGF has such a large molecular weight that does not pass through the blood-brain barrier, the development of an enhancing agent for NGF production that is capable of passing through the blood-brain barrier has been desired.

### DISCLOSURE OF INVENTION

In view of the above-mentioned situation, an object of the present invention is to provide a medicament for a disease requiring enhancement for NGF production upon treatment or prevention utilizing a safe enhancing agent for NGF production derived from natural product, and foodstuff and the like containing the above compound in a high content.

Summarizing the present invention, the first invention, the second invention, the third invention and the fourth invention of the present invention relate to a novel compound represented by the following general formula (I): 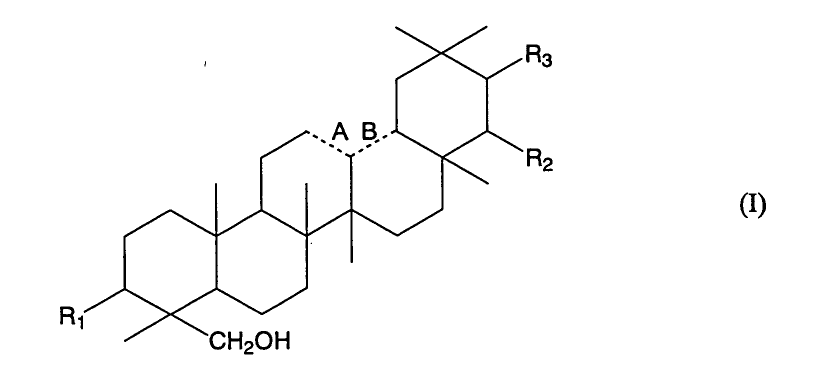 wherein of the bond lines indicated by dotted lines, when A is a double bond and B is a single bond, R₁ is -O-GlcUA-Gal-Glc, R₂ is -O-Ara-2-AcXyl, -O-Ara-3-AcXyl, -O-Ara-4-AcXyl, -O-Ara-2,3-diAcXyl, -O-Ara-2,4-diAcXyl, -O-Ara-3,4-diAcXyl or -O-Ara-3,4,6-triAcGlc, and R₃ is -OH; alternatively,
when A is a single bond and B is a double bond, R₁ is -OH, R₂ is -OH and R₃ is -H, wherein GlcUA is glucuronic acid residue, Gal is galactose residue, Glc is glucose residue, Ara is arabinose residue, AcXyl is acetylated xylose residue, AcGlc is acetylated glucose residue,
or a salt thereof; a therapeutic agent or prophylactic agent for a disease showing sensitivity to the above compound, an enhancing agent for nerve growth factor production, and a food, beverage or feed for enhancing nerve growth factor production, wherein each comprises the compound as an effective ingredient;.

In addition, the fifth invention, the sixth invention and the seventh invention of the present invention relate to a therapeutic agent or prophylactic agent requiring enhancement for nerve growth factor production upon treatment or prevention, an enhancing agent for nerve growth factor production, and a food, beverage or feed for enhancing nerve growth factor production characterized in that each comprises as an effective ingredient a compound selected from the group consisting of soyasaponin compounds, soyasapogenin compounds, glycyrrhizin and pharmacologically acceptable salts thereof.

In the embodiments of the fifth invention, the sixth invention and the seventh invention of the present invention, the soyasaponin compound and the soyasapogenin compound are exemplified by the compound represented by the following formula (II): 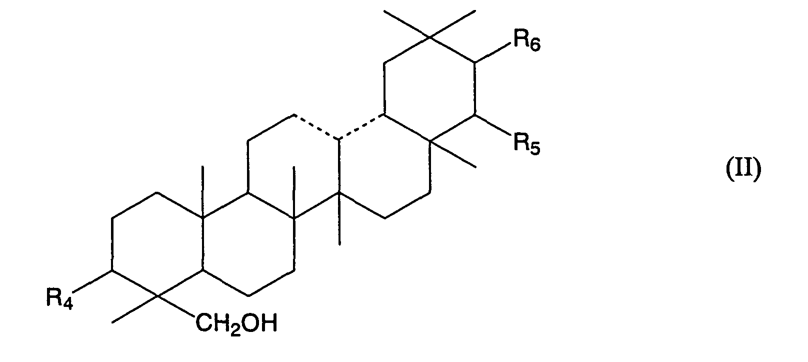 wherein the bond lines indicated by dotted lines are a single bond or a double bond; R₄ is -OH or -O- saccharide residue; R₅ is -OH, =O or -O-saccharide residue; R₆ is -OH or -H.

### BRIEF DESCRIPTION OF THE DRAWINGS

Figure 1 is a chart showing mass spectrum of the fraction 7 derived from the water extract of soybean embryo.
Figure 2 is a chart showing ¹H-NMR spectrum of the fraction 7 derived from the water extract of soybean embryo.
Figure 3 is a chart showing ¹³C-NMR spectrum of the fraction 7 derived from the water extract of soybean embryo.
Figure 4 is a chart showing mass spectrum of the fraction 9 derived from the water extract of soybean embryo.
Figure 5 is a chart showing ¹H-NMR spectrum of the fraction 9 derived from the water extract of soybean embryo.
Figure 6 is a chart showing ¹³C-NMR spectrum of the fraction 9 derived from the water extract of soybean embryo.
Figure 7 is a chart showing mass spectrum of the fraction 10 derived from the water extract of soybean embryo.
Figure 8 is a chart showing ¹H-NMR spectrum of the fraction 10 derived from the water extract of soybean embryo.
Figure 9 is a chart showing ¹³C-NMR spectrum of the fraction 10 derived from the water extract of soybean embryo.
Figure 10 is a chart showing mass spectrum of the fraction 12 derived from the water extract of soybean embryo.
Figure 11 is a chart showing ¹H-NMR spectrum of the fraction 12 derived from the water extract of soybean embryo.
Figure 12 is a chart showing ¹³C-NMR spectrum of the fraction 12 derived from the water extract of soybean embryo.
Figure 13 is a chart showing mass spectrum of the fraction 13 derived from the water extract of soybean embryo.
Figure 14 is a chart showing ¹H-NMR spectrum of the fraction 13 derived from the water extract of soybean embryo.
Figure 15 is a chart showing ¹³C-NMR spectrum of the fraction 13 derived from the water extract of soybean embryo.
Figure 16 is a chart showing mass spectrum of the fraction 15 derived from the water extract of soybean embryo.
Figure 17 is a chart showing ¹H-NMR spectrum of the fraction 15 derived from the water extract of soybean embryo.
Figure 18 is a chart showing ¹³C-NMR spectrum of the fraction 15 derived from the water extract of soybean embryo.
Figure 19 is a chart showing mass spectrum of the fraction 16 derived from the water extract of soybean embryo.
Figure 20 is a chart showing ¹H-NMR spectrum of the fraction 16 derived from the water extract of soybean embryo.
Figure 21 is a chart showing ¹³C-NMR spectrum of the fraction 16 derived from the water extract of soybean embryo.
Figure 22 is a chart showing mass spectrum of the fraction **a** derived from the acid hydrolyzate of soybean embryo.
Figure 23 is a chart showing ¹H-NMR spectrum of the fraction **a** derived from the acid hydrolyzate of soybean embryo.
Figure 24 is a chart showing ¹³C-NMR spectrum of the fraction **a** derived from the acid hydrolyzate of soybean embryo.

### BEST MODE FOR CARRYING OUT THE INVENTION

The present inventors have found that a specified saponin compound, a specified sapogenin compound and/or a salt thereof is useful as an enhancing agent for NGF production, derived from an edible plant, and the present invention has been accomplished thereby. According to the present invention, there could be provided a medicament containing as an effective ingredient these saponin compound, sapogenin compound and/or a pharmacologically acceptable salt thereof, foodstuff, a feed or the like, each containing the saponin compound, the sapogenin compound and/or a salt thereof in a high content.

The terms "enhancing action for NGF production" and "enhancing activity for NGF production" as used herein each refers to enhancement of NGF production and a function for enhancing NGF production, and is not intended to particularly strictly distinguish in its meaning. In addition, the term "enhance" encompasses an embodiment in which the amount of the desired substance is increased after the action as compared to that before the action of the effective ingredient of the present invention, as well as an embodiment (induction) in which the desired substance is produced by the action of the effective ingredient of the present invention. In addition, any of the substances listed as the effective ingredient in the present specification can be used alone or in admixture of two or more kinds in the present invention.

The saponin compound, the sapogenin compound and/or a salt thereof (hereinafter referred to as "compound of the present invention" in some cases), usable as the effective ingredient in the present invention may be those having an enhancing action for NGF production, which may be those derived from natural products, or may be synthesized products or semi-synthesized products. Those derived from natural product is preferably those derived from an edible plant, and those derived from an edible plant are exemplified by soyasaponin compounds or soyasapogenin compounds derived from beans such as soybeans. Also, there may be exemplified glycyrrhizin which is known as a saponin derived from *glycyrrhiza*.

The soybeans include four groups of saponins of A, B, E and DDMP saponins depending upon their chemical structures. The group A saponin is a saponin in which olean-12-en-3β,21β,22β,24-tetraol (soyasapogenol A) constitutes the aglycon; the group B saponin is a saponin in which olean-12-en-3β,22β,24triol (soyasapogenol B) constitutes the aglycon; further the group E saponin is a saponin in which olean-12-en-3β,24-diol-22-one (soyasapogenol E) constitutes the aglycon; and the DDMP saponin is a saponin in which the group B saponin constitutes the aglycon provided that 2,3-dihydro-2,5-dihydroxy-6-methyl-4H-pyran-4-one is bound at C-22 position *(Shokuhin to Kaihatsu (Food and Development),* Vol. 34, No. 7, pages 8-11, (1999)). In other words, in the present invention, as the soyasaponin compounds, the group A, B and E saponins and the DDMP saponin are especially suitably used. In addition, the compound of the present invention is exemplified by the compound represented by the above-mentioned general formula (II), especially preferably compounds a to n represented by the following general formula (III). 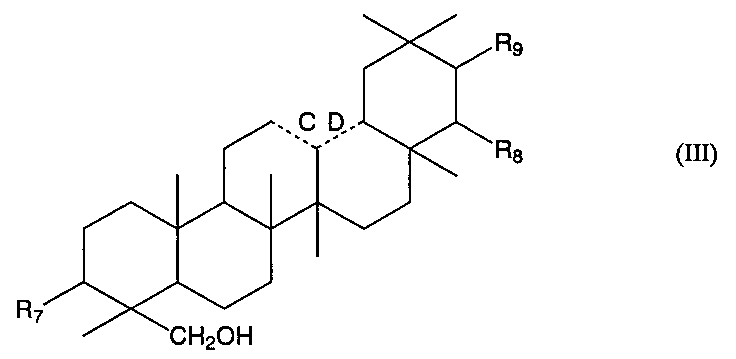 wherein the bond lines C and D indicated by dotted lines, and R₇, R₈, and R₉ are shown in Table 1.

**Table 1**

| | C | D | R₇ | R₈ | R₉ |
|---|---|---|---|---|---|
| Compound **a** | Double bond | Single bond | -O-GlcUA-Gal-Glc | -O-Ara-2-AcXyl | -OH |
| Compound **b** | Double bond | Single bond | -O-GlcUA-Gal-Glc | -O-Ara-3-AcXyl | -OH |
| Compound **c** | Double bond | Single bond | -O-GlcUA-Gal-Glc | -O-Ara-4-AcXyl | -OH |
| Compound **d** | Double bond | Single bond | -O-GlcUA-Gal-Glc | -O-Ara-2,3-diAcXyl | -OH |
| Compound **e** | Double bond | Single bond | -O-GlcUA-Gal-Glc | -O-Ara-2,4-diAcXyl | -OH |
| Compound **f** | Double bond | Single bond | -O-GlcUA-Gal-Glc | -O-Ara-3,4-diAcXyl | -OH |
| Compound **g** | Double bond | Single bond | -O-GlcUA-Gal-Glc | -O-Ara-3,4,6-triAcGlc | -OH |
| Compound **h** | Double bond | Single bond | -O-GlcUA-Gal-Glc | -O-Ara-2,3,4,6-tetraAcGlc | -OH |
| Compound **i** | Double bond | Single bond | -O-GlcUA-Gal-Glc | -OH | -H |
| Compound **j** | Double bond | Single bond | -O-GlcUA-Gal-Rham | -OH | -H |
| Compound **k** | Double bond | Single bond | -O-GlcUA-Gal-Glc | =O | -H |
| Compound **l** | Double bond | Single bond | -O-GlcUA-Gal-Rham | =O | -H |
| Compound **m** | Double bond | Single bond | -O-GlcUA-Gal-Glc | -O-Ara-2,3,4-triAcXyl | -H |
| Compound **n** | Single bond | Double bond | -OH | -OH | -H |

Here, the above-mentioned compounds a to g and n, specifically the compounds represented by the above-mentioned general formula (I), are compound obtained for the first time according to the present invention, of which enhancing actions for NGF production have been elucidated. In the present invention, there are also provided the compound, and a therapeutic agent or prophylactic agent of a disease showing sensitivity to the compound, comprising the compound as an effective ingredient.

In addition, as the compound of the present invention, those derived from soybeans and *glycyrrhiza* are preferable. The compound of the present invention is not limited in derivations thereof, as long as the compound is a soyasaponin compound, a soyasapogenin compound or glycyrrhizin, for instance, the compound represented by the above-mentioned general formula (II), even if the compound is not derived from soybean. In the present specification, AcXyl means acetylated xylose residue, AcGlc means acetylated glucose residue, and Rham means rhamnose residue. Also, the soyasapogenin compound means aglycon or isomer thereof without a saccharide residue in the soyasaponin compound.

The sugar constituting the saccharide residue may be a monosaccharide or a sugar chain, and the saccharide residue may comprise one or more saccharides. For instance, the sugar is exemplified by, for instance, glucose, glucuronic acid, xylose, rhamnose, galactose, threose, ribose, apiose, allose, arabinose, arabinopyranose, ribulose, mannose, talose, fucose, fructose, galacturonic acid, and sugar chains constituted by these saccharides. The number of the saccharides in the sugar chain is, but not particularly limited to, especially preferably from 2 to 5, from the viewpoint of exhibiting the desired effects of the present invention. Also, these saccharides may be subjected to a modification such as acetylation or esterification.

Furthermore, the compound of the present invention can be formed into a derivative (prodrug), which can be readily hydrolyzed in the body to exhibit the desired effects, by, for instance, subjecting the compound to esterification. The prodrug may be prepared in accordance with a known method. Here, the derivative may be their salts.

In addition, in the compound of the present invention, as the salts, pharmacologically acceptable salts are preferable. The salts used in the present invention are exemplified by, for instance, alkali metal salts, alkaline earth metal salts, salts with an organic base, and the like. The pharmacologically acceptable salt used in the present invention is referred to a salt of a compound which is substantially nontoxic against an organism, and has an enhancing action for NGF production. The salts of the compound as the effective ingredient of the present invention include, for instance, salts with sodium, potassium, calcium, magnesium, ammonium or protonated benzathine (N,N'-di-benzylethylenediamine), choline, ethanolamine, diethanolamine, ethylenediamine, meglamine (N-methylglucamine), benethamine (N-benzylphenetylamine), piperazine or tolomethamine (2-amino-2-hydroxymethyl-1,3-propanediol).

Also, various isomers such as optical isomers, keto-enol tautomers, and geometric isomers of the compound of the present invention can be all used in the present invention, even when each of isomers is isolated, as long as these isomers have an enhancing activity for NGF production. Therefore, the compound of the present invention encompasses derivatives thereof, isomers thereof and salts thereof, as long as the desired effects of the present invention can be obtained.

If the compound of the present invention is commercially available, such a compound can be utilized. Also, the compound of the present invention can be prepared. The preparation process includes a process comprising extracting and purifying the compound from a plant to give the compound in accordance with a conventional method.

The compound of the present invention can be prepared from a natural product by combining known preparation processes. For instance, the compound can be extracted and purified from a substance containing a soyasaponin compound, a soyasapogenin compound or a salt thereof, for instance, a plant such as soybean. In addition, when soybean is used, soybean embryo and cotyledon are preferably used as raw materials. As the extraction solvent, there can be used, for instance, a hydrophilic or lipophilic solvent such as water, chloroform, an alcohol such as ethanol, methanol or isopropyl alcohol, a ketone such as acetone or methyl ethyl ketone, methyl acetate or ethyl acetate, alone or as a liquid mixture. The extraction temperature and time may be appropriately set as desired, and the extraction procedures may be repeated several times as desired. As the purification means, a known purification means such as a chemical method or physical method may be employed. A conventionally known purification method such as gel filtration method, fractionation method with a molecular weight fractionation membrane, solvent extraction method, and various chromatographic methods using ion exchange resins and the like may be combined to concentrate, isolate or the like the desired compound to be used as the effective ingredient. The medicament or the like of the present invention described later can be prepared by using the concentrate of the compound, without having to use the isolated compound.

In addition, glycyrrhizin usable in the present invention is not particularly limited. Glycyrrhizin is commercially available, or can be obtained by extracting and purifying from *glycyrrhiza*. The extraction and purification of glycyrrhizin can be carried out in accordance with the method described for the above-mentioned soyasaponin compound or the like. Also, in the present invention, the glycyrrhizin derivative can be used.

When the compound usable as the effective ingredient in the present invention is obtained as a synthesized product or semi-synthesized product, the desired compound may be synthesized, for instance, in accordance with a known method.

Whether or not the desired compound is obtained can be confirmed by, for instance, determining the structure by mass spectroscopy, nuclear magnetic resonance method or the like as described in Examples set forth below.

According to the present invention, there is provided a therapeutic agent or prophylactic agent for a disease requiring enhancement of NGF production upon treatment or prevention, comprising as the effective ingredient the compound of the present invention as described above.

NGF is an endogenous growth factor for maintaining viability and functions of nerve cells, elongating nerve cells in accordance with a concentration gradient of NGF, or the like. By enhancing the production of NGF, the treatment or prevention of senile dementia such as Alzheimer's disease, peripheral nerve disorder, cerebrovascular disorder, cerebral tumor, cerebral apicitis, nerve degenerative disease caused by head injury, diseases requiring recovery and regeneration of nerve functions, caused by intoxication with an anesthetic or the like can be carried out. In addition, it is useful in the treatment or prevention of amyotrophic lateral sclerosis, drug-induced peripheral nerve disorder, diabetic peripheral nerve disorder, Parkinson's disease, sensory nerve disorder, retinitis pigmentosa, macular dystrophy, and the like. In other words, by administering or taking the medicament, or the foodstuff or feed mentioned later of the present invention, the treatment or prevention of the above-mentioned diseases can be carried out.

The medicament of the present invention encompasses a therapeutic agent or prophylactic agent, characterized in that the therapeutic agent or prophylactic agent comprises as the effective ingredient the compound represented by the general formula (I) and/or a pharmacologically acceptable salt thereof, which are obtained for the first time in the present invention, wherein the therapeutic agent or prophylactic agent is for a disease showing sensitivity to the above compound.

The above-mentioned therapeutic agent or prophylactic agent of the present invention can be prepared by combining the compound of the present invention, for instance, a soyasaponin compound, a soyasapogenin compound, glycyrrhizin or a pharmacologically acceptable salt thereof as an effective ingredient with a known pharmaceutical carrier to form into a preparation.

The content of the effective ingredient in the medicament of the present invention cannot be absolutely determined because the content differs depending upon the diseases to be treated or the like, use embodiments and the like. The content is usually from 0.01 to 50% by weight or so, preferably from 0.1 to 10% by weight or so.

In general, the therapeutic agent or prophylactic agent of the present invention is manufactured by formulating the compound of the present invention with a pharmacologically acceptable liquid or solid carrier, and optionally a solvent, a dispersant, an emulsifier, a buffer, a stabilizer, an excipient, a binder, a disintegrant, a lubricant, or the like can be added thereto, so that a solid agent such as a tablet, a granule, a powder, a fine powder, or a capsule, or a liquid agent such as a common liquid agent, a suspension agent or an emulsion agent can be formed. In addition, there can be also made into a dry product which can be made liquid by adding an appropriate liquid vehicle before use.

The therapeutic agent or prophylactic agent of the present invention can be administered in any of the forms of orally administered agents and non-orally administered agents such as injections and drops.

The dose of the therapeutic agent or prophylactic agent of the present invention is changeable and properly set depending upon its preparation form, administration method, purpose of use, and age, body weight, symptom of a patient to which the therapeutic agent or prophylactic agent is applied. Generally, the dose of the agent as the dose for human per day of the effective ingredient contained in the preparation is usually from 1 µg to 1 g/kg body weight, preferably from 10 µg to 200 mg/kg body weight. As a matter of course, the dose varies depending upon various conditions, so that an amount smaller than the dose mentioned above may be sufficient, or an amount exceeding the dose range may be required. Also, the period of administration is not particularly limited. The medicament of the present invention can be directly orally administered, or the agent can be added to any foodstuffs be taken on a daily basis.

In addition, the present invention can provide an enhancing agent for NGF production, comprising the compound of the present invention as the effective ingredient. The enhancing agent may be the above-mentioned effective ingredient itself, or a composition comprising the above-mentioned effective ingredient. In the embodiment of the present invention, the salt as the effective ingredient is preferably a pharmacologically acceptable salt. The enhancing agent for NGF production may be produced by, for instance, formulating the above-mentioned effective ingredient with other ingredients which can be used for the same application as the effective ingredient, and forming into a form of reagent usually used according to the above-mentioned process for producing the therapeutic agent or prophylactic agent. The content of the above-mentioned effective ingredient in the enhancing agent is not particularly limited, as long as the content is in an amount so that the desired effects of the present invention can be exhibited in consideration of administration method, purpose of use, or the like of the enhancing agent. The content of the effective ingredient of the present invention in the enhancing agent for NGF production is usually from 0.01 to 50% by weight or so, preferably from 0.1 to 10% by weight or so. Also, the amount of the enhancing agent used is not particularly limited, as long as the desired effects of the present invention can be exhibited. Especially in the case where the enhancing agent is administered to a living body, the enhancing agent is preferably used in an amount so that the effective ingredient can be administered within the dose range of the effective ingredient for the above-mentioned therapeutic agent or prophylactic agent. The enhancing agent for NGF production is useful for enhancement for NGF production in a disease requiring enhancement for NGF production. In addition, the enhancing agent is also useful for screening of drugs for diseases associated with NGF. Furthermore, the enhancing agent is useful for functional studies concerning NGF or physical changes in nerve cells.

Next, there is explained a food or beverage (hereinafter referred to as "foodstuff" in some cases) or a feed, each comprising the above-mentioned effective ingredient. In the foodstuff or feed of the present invention, the term "comprising" encompasses the meaning of containing, adding and diluting. Therefore, the foodstuff or feed of the present invention is a food, beverage or feed in which the compound of the present invention is contained, added and/or diluted. The foodstuff or the like is very useful in the amelioration of symptoms or prevention of a disease requiring enhancement for NGF production upon treatment or prevention. In addition, the foodstuff or the like is useful in the maintenance of homeostasis of a living body. The salt of the compound usable as the effective ingredient is preferably a pharmacologically acceptable salt.

As used herein in the foodstuff or feed of the present invention, the term "containing" refers to an embodiment of containing the effective ingredient usable in the present invention in the foodstuff or the like; the term "adding" refers to an embodiment of adding the effective ingredient usable in the present invention to a raw material for the foodstuff or the like; and the term "diluting" refers to an embodiment of adding a raw material for the foodstuff or the like to the effective ingredient usable in the present invention.

The process for preparing the food, beverage or feed of the present invention is not particularly limited. A generally used process for preparing a food, beverage or feed can be employed, as long as the resulting food, beverage or feed may contain the compound of the present invention as the effective ingredient in a high content, wherein the content is higher than that of a usual foodstuff or feed. In other words, the foodstuff or feed of the present invention can be prepared by concentrating or isolating the effective ingredient of the present invention from the raw materials, and formulating the effective ingredient in a known foodstuff or feed. The concentration or isolation encompasses the synthesis of the effective ingredient. Also, when the raw materials can be treated such that the content of the effective ingredient is increased in the raw materials, the resulting processed product can be directly used as the foodstuff of the present invention, as long as the raw materials may be used for edible purposes for human. For instance, the processed products include concentrated juice, vegetable juice and the like. The expression "contained ... in a high content" as used herein means that the weight of the compound of the present invention per unit weight of the food, beverage or feed of the present invention is larger than the weight of the compound of the present invention per unit weight of the raw material, for instance, soybean, for the effective ingredient of the present invention. Also, the weight refers to the weight of the solid content.

The food or beverage of the present invention is not particularly limited. The food or beverage includes, for instance, processed agricultural and forest products, processed stock raising products, processed marine products and the like, including processed grain products (for instance, processed wheat products, processed starch products, processed premix products, noodles, macaronis, bread, bean jam, buckwheat noodles, wheat-gluten bread, rice noodle, *fen-tiao*, packed rice cake and the like); processed fat and oil products (for instance, plastic fat and oil, tempura oil, salad oil, mayonnaise, dressing and the like); processed soybean products (for instance, tofu products, soybean paste, fermented soybeans and the like); processed meat products (for instance, ham, bacon, pressed ham, sausage and the like); marine products (for instance, frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, preserved food boiled down in soy sauce (*tsukudani*) and the like); milk products (for instance, raw material milk, cream, yogurt, butter, cheese, condensed milk, powder milk, ice cream and the like); processed vegetable and fruit products (for instance, paste, jam, pickled vegetables, fruit beverages, vegetable beverages, mixed beverages and the like); confectionaries (for instance, chocolates, biscuits, sweet bun, cake, rice cake snacks, rice snacks and the like); alcohol beverages (for instance, *sake*, Chinese liquor, wine, whiskey, Japanese distilled liquor *(shochu),* vodka, brandy, gin, rum, beer, refreshing alcoholic beverages, fruit liquor, liqueur and the like); luxury drinks (for instance, green tea, tea, oolong tea, coffee, refreshing beverages, lactic acid beverages and the like); seasonings (for instance, soy sauce, sauce, vinegar, sweet rice wine and the like); canned, binned or pouched foods (for instance, rice topped cooked beef and vegetable, rice boiled together with meat and vegetables in a small pot, steamed rice with red beans, curry roux and rice, other precooked foods and the like); semi-dry or concentrated foods (for instance, liver pastes and other spreads, soups for buckwheat noodles or wheat noodles, concentrated soups and the like); dry foods (for instance, instant noodles, instant curry roux, instant coffee, powder juice, powder soup, instant soybean paste (*miso*) soup, precooked foods, precooked beverages, precooked soup and the like); frozen foods (for instance, *sukiyaki*, pot-steamed hotchpotch, split and grilled eel, hamburger steak, *shao-mai*, dumpling stuffed with minced pork, various sticks, fruit cocktails and the like); solid foods; liquid foods (for instance, soups and the like); spices; and the like.

The content of the effective ingredient in the foodstuff of the present invention is not particularly limited, and the content is usually from 0.01 to 50% by weight or so, preferably from 0.1 to 10% by weight or so.

The feed of the present invention is, for instance, a feed for cattle, culture fish, poultry, or pet, which means an artificial food or beverage for an organism other than human, comprising the compound of the present invention. The beverage includes drinking water for cattle and the like. In addition, one embodiment of the present invention provides a feed additive for enhancement of NGF production, comprising the effective ingredient of the present invention. The additive is used by adding to an ordinary feed or water in which an organism is immersed (water for culturing a culture fish, or the like).

The content of the effective ingredient in the feed of the present invention is not particularly limited, and the content is usually from 0.01 to 50% by weight or so, preferably from 0.1 to 10% by weight or so. The content of the effective ingredient in the above-mentioned additive is not particularly limited, and may be properly adjusted so as to give the desired effects according to its purposes.

The form of the feed (including the above-mentioned additive) of the present invention is not particularly limited, and may be in the form of a powder, a liquid or a solid. Here, the salt of the compound usable as the effective ingredient is preferably a pharmacologically acceptable salt.

In the present invention, the content of the compound of the present invention in the medicament, foodstuff or feed is as mentioned above. The compound is contained in a high content which is equal to or higher than the content of the soyasaponin compound, the soyasapogenin compound and glycyrrhizin in an ordinary foodstuff, as long as the content is such an amount that NGF production is enhanced in a living body upon administration, intake or the like.

No toxicity is found in the compound of the present invention, which is the effective ingredient. For instance, no toxicity is found when a mouse (male, 6-week old) is orally administered with the saponin compound, glycyrrhizin or a salt thereof usable in the present invention at 1000 mg per kg body weight.

In addition, the present invention also provides a novel soyasaponin compound and a novel soyasapogenin compound, which are represented by the above-mentioned general formula (I), wherein each of the compounds has an enhancing action for NGF production. The process for the preparation is as described above, and is described in further detail by means of the following Examples. The novel compounds of the present invention can be prepared in reference to the Examples, without being limited thereto.

### EXAMPLES

The present invention will be more concretely described hereinbelow by means of the examples, without by no means limiting the scope of the present invention thereto.

### Example 1 Fractionation of Fraction Derived from Water Extract of Soybean Embryo

(1) Twenty-five liters of distilled water was added to 9.6 kg of dry soybean embryo, and the soybean embryo was immersed for one hour, and thereafter disrupted with a blender. Next, thereto was added 40 liters of distilled water, and the mixture was stirred for 2 hours. Thereafter, centrifugation was carried out at 5000 g for 7 minutes, to give a precipitate 1 and supernatant 1 (54 liters). Forty liters of distilled water was added to the precipitate 1, and the mixture was stirred for 2 hours. Thereafter, centrifugation was carried out at 5000 g for 7 minutes, to give a precipitate 2 (19.7 kg) and supernatant 2 (36 liters). The supernatant 1 and the supernatant 2 were mixed together, to give 90 L of a water extract of soybean embryo.
(2) Thirty-nine liters of ethanol was added to 90 liters of the water extract of soybean embryo obtained in item (1) of Example 1, and the mixture was stirred. Thereafter, centrifugation was carried out at 5000 g for 7 minutes, to give a precipitate 3 and supernatant 3 (90 liters). The supernatant 3 was concentrated to a volume of 18 liters with a rotary evaporator, an equal volume of ethanol was added thereto, and the mixture was stirred. Thereafter, centrifugation was carried out at 5000 g for 7 minutes, to give a precipitate 4 and supernatant 4 (15 liters). The supernatant 4 was concentrated to give 1.7 liters of a concentrate of water extract of soybean embryo.
(3) A mixture prepared by adding 500 mL of distilled water to 110 mL of the concentrate of water extract of soybean embryo obtained in item (2) of Example 1 was fractionated using reverse phase chromatography. The resin used was Cosmosil 140 C18-OPN (manufactured by nakalai tesque, amount of resin: 300 mL). The elution and fractionation were carried out using 1500 mL of distilled water, a 30% aqueous ethanol solution, a 50% aqueous ethanol solution, and 100% ethanol in that order as the developing solvents, to give each of Cosmosil-eluted fractions derived from the water extract of soybean embryo.
(4) The Cosmosil-30% ethanol-eluted fraction derived from the water extract of soybean embryo obtained in item (3) of Example 1 was concentrated under reduced pressure. Thereafter, acetone was added to the concentrate in an amount three times the volume of the concentrate, and the mixture was stirred, and centrifugation was carried out at 5000 g for 7 minutes, to give a precipitate 5 and supernatant 5.
(5) The supernatant 5 obtained in item (4) of Example 1 was concentrated to dryness, and thereafter the residue was dissolved in ethyl acetate : acetic acid : water (volume ratio, hereinafter the same) = 8 : 3: 2 (10 mL). The mixture was fractionated using silica chromatography. The conditions therefor are given below. BW-300SP (manufactured by Fuji Silysia Chemical Ltd.; amount of resin: 300 mL) was used as the silica gel. The elution was carried out using ethyl acetate : acetic acid : water = 8 : 3 : 2 (1000 mL) and ethanol: water = 5 : 1 (500 mL) in that order as the developing solvents, to give eluted fractions of a fraction 0 (300 mL), a fraction 1 (200 mL), a fraction 2 (200 mL), a fraction 3 (100 mL), a fraction 4 (150 mL), a fraction 5 (200 mL) and a fraction 6 (120 mL) in that order. Each of the eluates was concentrated under reduced pressure, to give each of silica column fractions 0 to 6 derived from the water extract of soybean embryo.
(6) The silica column fraction 5 derived from the water extract of soybean embryo obtained in item (5) of Example 1 was dissolved in 60 mL of distilled water. Thereafter, the solution was fractionated using reverse phase chromatography. The conditions therefor are given below. TSK gel ODS-80Ts (21.5 mm × 30 cm; manufactured by Tosoh Corporation) was used as a column. The elution ratio of Solvent A (mixture prepared by mixing distilled water and acetonitrile in a volume ratio of 1 : 3) to Solvent B (mixture prepared by mixing distilled water and acetonitrile in a volume ratio of 3 : 1) was such that the ratio of Solvent A was retained at 100% from 0 to 10 minutes, the ratio of Solvent B increased linearly from 0 to 30% from 10 to 25 minutes, the ratio of Solvent B increased linearly from 30 to 100% from the next 25 to 40 minutes, and the ratio of Solvent B was retained at 100% for the subsequent 15 minutes. The elution rate was 5 mL/minute, and the detection was carried out at 215 nm. The fractions 1 to 21 derived from the water extract of soybean embryo were fractionated using ultraviolet absorption of an eluate as an index.

### Example 2 Enhancing Activity for NGF Production of 3-O-[β-D-glucopyranosyl(1→2)-β-D-galactopyranosyl-(1→2)-β-D-glucuronopyranosyl]-22-O-[2-O-acetyl-β-D-xylopyranosyl(1→3)-α-L-arabinopyranosyl]soyasapogenol A

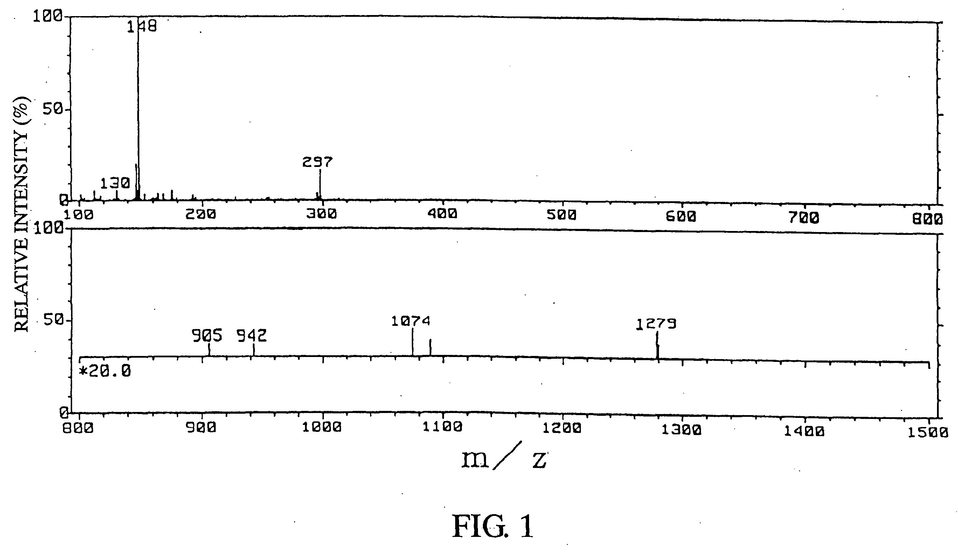

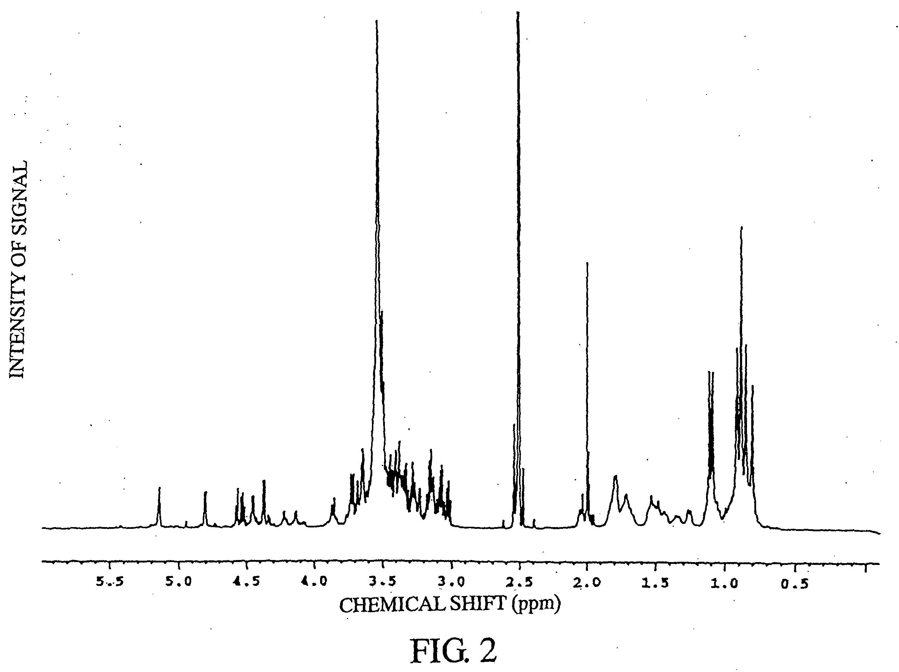

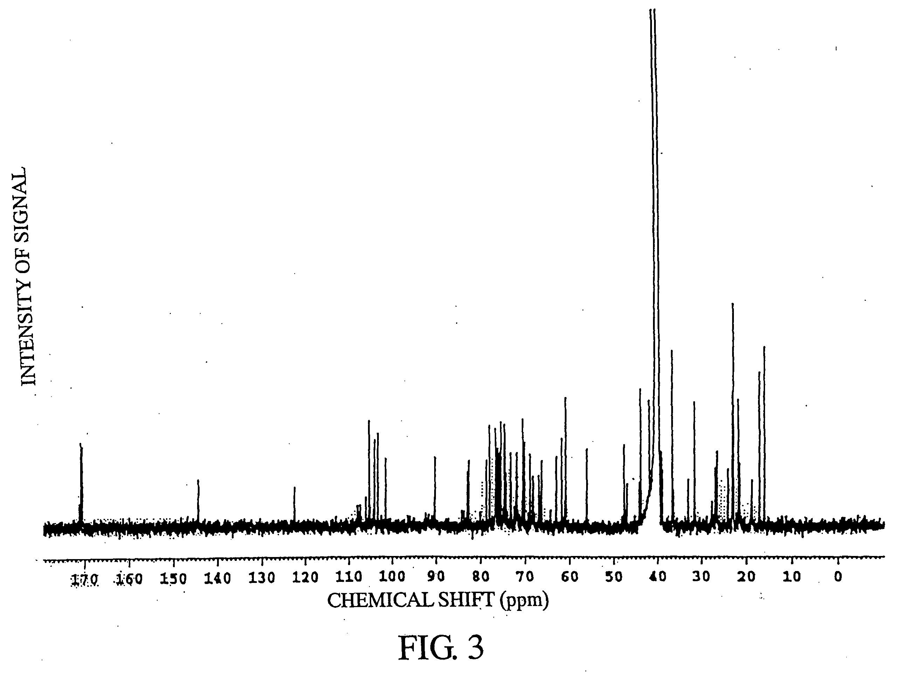

(1) The mass spectrum (MS) of the fraction 7 derived from the water extract of soybean embryo (fraction including a peak detected at a retention time of 32.4 minutes) fractionated in item (6) of Example 1 was measured with a mass spectrometer (DX302, manufactured by JEOL LTD.) by FAB-MS technique. As the matrix, triethanolamine was used. As a result, a peak of m/z 1279 (M-H)⁻ was detected. Figure 1 shows the mass spectrum of the fraction 7 derived from the water extract of soybean embryo. In Figure 1, the axis of abscissas is m/z value, and the axis of ordinates is relative intensity.
   The structure of the fraction 7 derived from the water extract of soybean embryo was analyzed by measuring various NMR spectra using nuclear magnetic resonance (NMR) spectrometer (Model AVANCE600, manufactured by Bruker Biospin K.K.). The signals of NMR are shown below.
   ¹H-NMR:
   sapogenol moiety; δ3.28 (1H, m, 3-H), 5.15 (1H, br-s, 12-H), 3.45 (1H, m, 21-H),
   3.23 (1H, br-s, 22-H), 3.14 (1H, m, 24-H), 3.85 (1H, m, 24-H)
   3-*O*-β-D-glucronopyranosyl moiety; δ4.45 (1H, d, J=7.2 Hz, 1'-H),
   3.44 (1H, m, 2'-H), 3.57 (1H, m, 3'-H), 3.32 (1H, m, 4'-H), 3.65 (1H, m, 5'-H)
   2'-*O*-β-D-galactopyranosyl moiety; δ4.81 (1H, d, J=6.6 Hz, 1"-H),
   3.51 (1H, m, 2"-H), 3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.38 (1H, d, J=7.8 Hz, 1"'-H),
   3.02 (1H, m, 2"'-H), 3.17 (1H, m, 3"'-H), 3.08 (1H, m, 4"'-H),
   3.14 (1H, m, 5"'-H), 3.46 (1H, m, 6"'-H), 3.73 (1H, m, 6"'-H)
   22-*O*-α-L-arabinopyranosyl moiety; δ4.13 (1H, m, 1""-H), 3.42 (1H, m, 2""-H),
   3.42 (1H, m, 3""-H), 3.69 (1H, m, 4""-H), 3.35 (1H, m, 5""-H),
   3.62 (1H, m, 5""-H)
   3""-*O*-β-D-xylopyranosyl moiety; δ4.57 (1H, d, J=7.8 Hz, 1""'-H),
   4.52 (1H, m, 2""'-H), 3.29 (1H, m, 3""'-H), 3.37 (1H, m, 4""'-H),
   3.09 (1H, m, 5""'-H), 3.72 (1H, m, 5""'-H), 2.00 (3H, s, 2""'-CH₃)
   Here, in ¹H-NMR, the sample was dissolved in deuterated dimethyl sulfoxide, and the chemical shift of the residual proton of deutrated dimethyl sulfoxide was expressed as 2.51 ppm. Figure 2 shows ¹H-NMR spectrum of the fraction 7 derived from the water extract of soybean embryo. In Figure 2, the axis of abscissas is chemical shift, and the axis of ordinates is intensity of signal.
   ¹³C-NMR:
   sapogenol moiety; δ 90.3 (3-C), 122.5 (12-C), 144.4 (13-C), 74.7 (21-C), 92.0 (22-C), 63.0 (24-C)
   3-*O*-β-D-glucronopyranosyl moiety; δ104.1 (1'-C), 79.0 (2'-C), 76.6 (3'-C), 71.9 (4'-C), 76.2 (5'-C), 171.0 (6'-C)
   2'-*O*-β-D-galactopyranosyl moiety; δ101.6 (1"-C), 82.7 (2"-C), 73.4 (3"-C), 69.0 (4"-C), 75.5 (5"-C), 60.9 (6"-C)
   2"-*O*-β-D-glucopyranosyl moiety; δ105.3 (1"'-C), 75.5 (2"'-C), 76.7 (3"'-C), 70.6 (4"'-C), 78.1 (5"'-C), 61.8 (6"'-C)
   22-*O*-α-L-arabinopyranosyl moiety; δ108.0 (1""-C), 71.9 (2""-C), 83.0 (3""-C), 68.5 (4""-C), 67.0 (5""-C)
   3""-*O*-β-D-xylopyranosyl moiety; δ103.3 (1""'-C), 74.6 (2""'-C), 74.7(3""'-C), 70.2 (4""'-C), 65.3 (5""'-C), 170.5 (2""'-C=O), 21.8 (2""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 3 shows ¹³C-NMR spectrum of the fraction 7 derived from the water extract of soybean embryo. In Figure 3, the axis of abscissas is chemical shift, and the axis of ordinates is intensity of signal.
   The fraction 7 derived from the water extract of soybean embryo was mixed with 1 N H₂SO₄ (solution having a volume ratio of dioxane and water of 1 : 3), and the mixture was heated at 80°C for 4 hours, to produce a saccharide component and a non-saccharide component. The non-saccharide component was subjected to thin layer chromatography (Silica gel 60 F254 manufactured by Merck, developing solvent A: prepared by mixing chloroform and methanol in a ratio of 10 : 1). As a result, a spot having the same Rf values as in Soyasapogenol A was detected. The saccharide component was subjected to thin layer chromatography (developing solvent B: prepared by mixing water and acetonitrile in a ratio of 3 : 17). As a result, arabinose, glucose, xylose and galactose were detected.
   From the above analyses of the mass spectrum and NMR spectrum and the analyses of the non-saccharide component and the saccharide component after acid hydrolysis performed for the fraction 7 derived from the water extract of soybean embryo, it was identified that the fraction 7 derived from the water extract of soybean embryo is 3-*O*-[β-D-glucopyranosyl(1→2)-β-D-galactopyranosyl-(1→2)-β-D-glucuronopyranosyl]-22-*O*-[2-*O*-acetyl-β-D-xylopyranosyl(1→3)-α-L-arabinopyranosyl]soyasapogenol A (molecular weight: 1280). The compound is a compound **a** in Table 1 mentioned above.
(2) The enhancing activity for NGF production of the compound a was assayed. L-M cells (ATCC CCL-1.2) from murine fibroblasts were suspended in an M199 medium (manufactured by Bio Whittaker) containing 0.5% bactopeptone (manufactured by Gibco) so as to have a concentration of 1.5 × 10⁵ cells/mL. The suspension was put in a 96 well plate in an amount of 0.1 mL each well, and the cells were aseptically cultured. After culturing the cells for 3 days, the medium was removed therefrom, and exchanged with an M199 medium containing 0.5% bovine serum albumin (manufactured by Sigma). The compound **a** was added thereto, and the cells were cultured for 20 hours. After the termination of the culture, the concentration of NGF in the culture medium was assayed by an enzyme immunoassay method (NGF Emax Immuno Assay System, manufactured by Promega). The added amount, as expressed in the final concentration, is as shown in Table 2. The experiment was carried out twice, and an average value was taken. As a result, it was clarified that the compound a has an enhancing activity for NGF production. The results are shown in Table 2. The amount of NGF production of the control was 0.739 ng/mL. As to the control, the concentration in the culture medium was determined in the same manner except that the compound a was not added. It is the same as that of the following examples.

### Example 3 Enhancing Activity for NGF Production of 3-O-[β-D-glucopyranosyl(1→2)-β-D-galactopyranosyl-(1→2)-β-D-glucuronopyranosyl]-22-O-[3-O-acetyl-β-D-xylopyranosyl(1→3)-α-L-arabinopyranosyl]soyasapogenol A

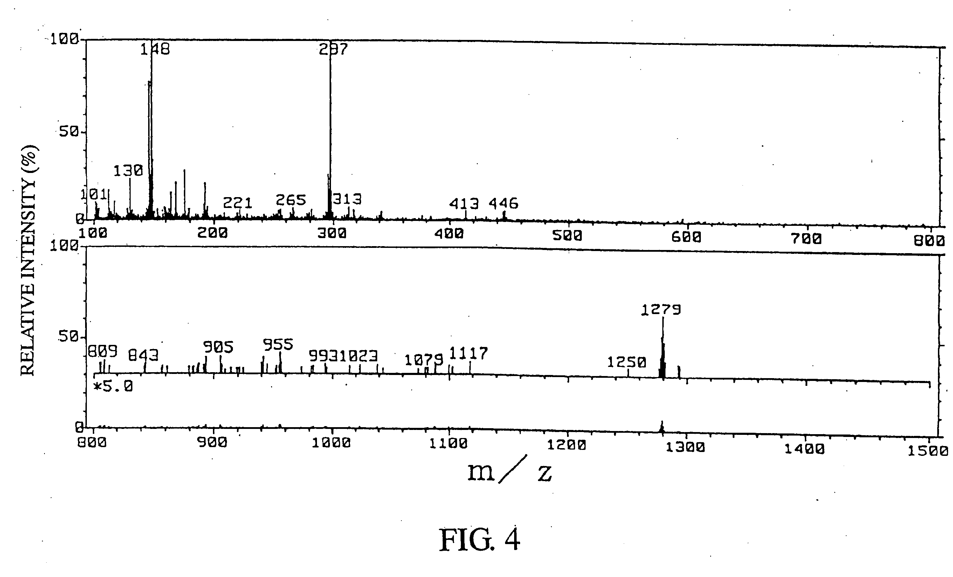

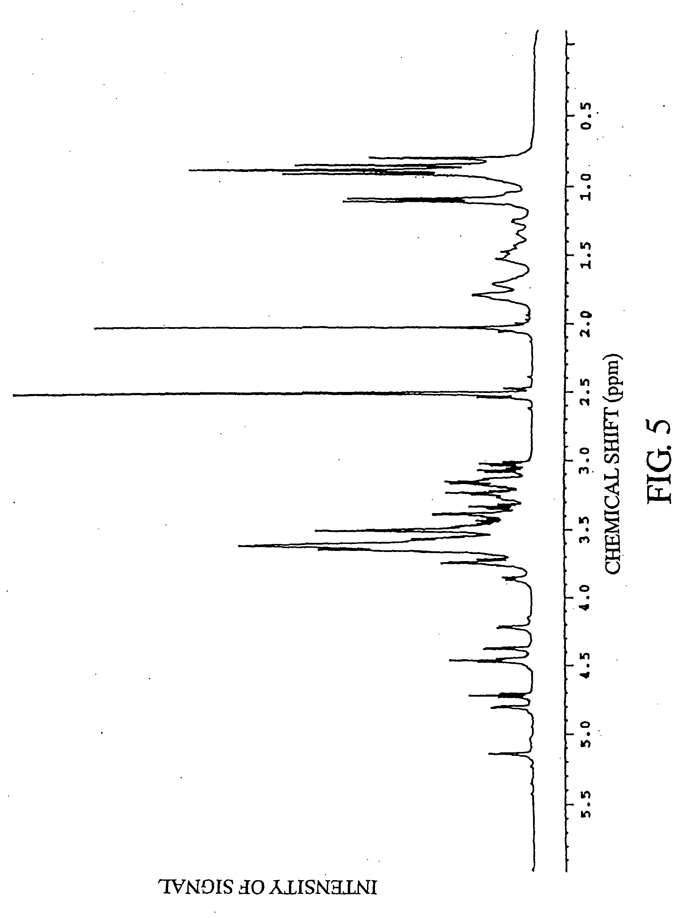

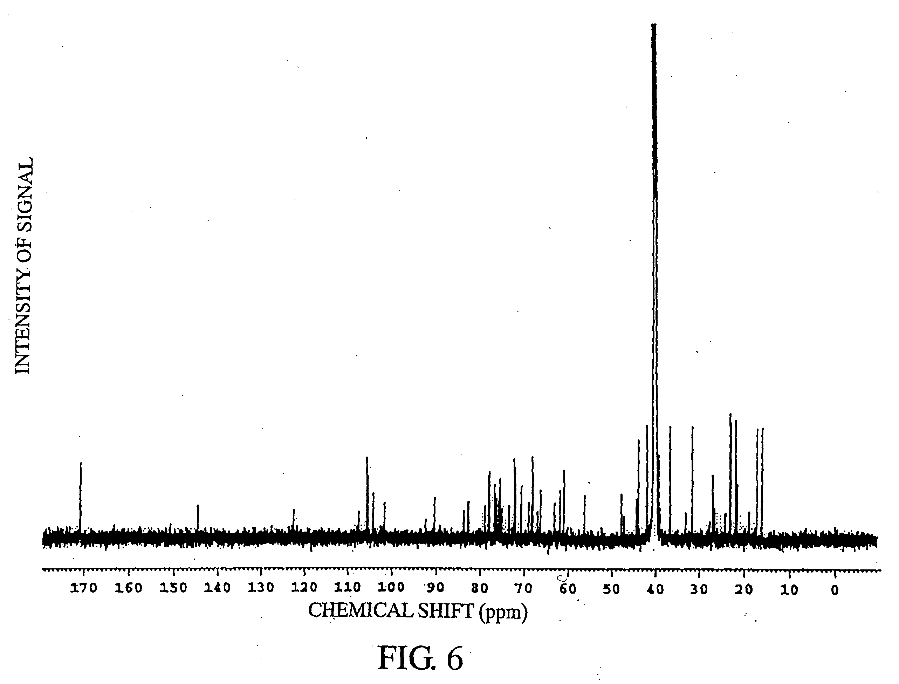

(1) The mass spectrum of the fraction 9 derived from the water extract of soybean embryo (fraction including a peak detected at a retention time of 34.2 minutes) fractionated in item (6) of Example 1 was measured in the same manner as in item (1) of Example 2. As the matrix, triethanolamine was used. According to mass spectrometry, a peak of m/z 1279 (M-H)- was detected. Figure 4 shows the mass spectrum of the fraction 9 derived from the water extract of soybean embryo. In Figure 4, the axis of abscissas is m/z value, and the axis of ordinates is relative intensity.
   The NMR spectrum of the fraction 9 derived from the water extract of soybean embryo was measured in the same manner as in item (1) of Example 2. The signals of NMR are shown below.
   ¹H-NMR:
   sapogenol moiety; δ3.27 (1H, m, 3-H), 5.14 (1H, br-s, 12-H), 3.39 (1H, m, 21-H),
   3.24 (1H, br-s, 22-H), 3.14 (1H, m, 24-H), 3.86 (1H, m, 24-H)
   3-*O*-β-D-glucronopyranosyl moiety; δ4.45 (1H, m, 1'-H), 3.43 (1H, m, 2'-H),
   3.57 (1H, m, 3'-H), 3.33 (1H, t, J=9.7 Hz, 4'-H), 3.65 (1H, m, 5'-H)
   2'-*O*-β-D-galactopyranosyl moiety; δ4.80 (1H, d, J=6.6 Hz, 1"-H),
   3.51 (1H, m, 2"-H), 3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.37 (1H, d, J=7.8 Hz, 1"'-H),
   3.03 (1H, dd, J=7.8, 9.0 Hz, 2"'-H), 3.17 (1H, t, J=9.0 Hz, 3"'-H),
   3.07 (1H, t, J=9.0 Hz, 4"'-H), 3.15 (1H, m, 5"'-H), 3.47 (1H, m,6"'-H), 3.73 (1H, m, 6"'-H)
   22-*O*-α-L-arabinopyranosyl moiety; δ4.22 (1H, d, J=7.8 Hz, 1""-H),
   3.55 (1H, m, 2""-H), 3.51 (1H, m, 3""-H), 3.75 (1H, m, 4""-H),
   3.40 (1H, m, 5""-H), 3.63 (1H, m, 5""-H)
   3""-*O*-β-D-xylopyranosyl moiety; δ4.47 (1H, m, 1""'-H), 3.23 (1H, m,2""'-H),
   4.72 (1H, t, J=9.3 Hz, 3""'-H), 3.48 (1H, m, 4""'-H), 3.18 (1H, m, 5""'-H),
   3.74 (1H, m, 5""'-H), 2.03 (3H, s, 3""'-CH₃)
   Here, in ¹H-NMR, the sample was dissolved in deuterated dimethyl sulfoxide, and the chemical shift of the residual proton of deuterated dimethyl sulfoxide was expressed as 2.51 ppm. Figure 5 shows ¹H-NMR spectrum of the fraction 9 derived from the water extract of soybean embryo. In Figure 5, the axis of abscissas is chemical shift, and the axis of ordinates is intensity of signal.
   ¹³C-NMR:
   sapogenol moiety; δ 90.4 (3-C), 122.5 (12-C), 144.4 (13-C), 75.0 (21-C), 92.3 (22-C), 63.0 (24-C)
   3-*O*-β-D-glucronopyranosyl moiety; δ104.1 (1'-C), 78.9 (2'-C), 76.7 (3'-C), 72.0 (4'-C), 76.1 (5'-C), 171.0 (6'-C)
   2'-*O*-β-D-galactopyranosyl moiety; δ101.6 (1"-C), 82.7 (2"-C),73.4 (3"-C), 69.0 (4"-C), 75.7 (5"-C), 61.0 (6"-C)
   2"-*O*-β-D-glucopyranosyl moiety; δ105.3 (1"'-C), 75.5 (2"'-C), 76.8 (3"'-C), 70.6 (4"'-C), 78.1 (5"'-C), 61.8 (6"'-C)
   22-*O*-α-L-arabinopyranosyl moiety; δ107.4 (1""-C), 71.9 (2""-C), 83.7 (3""-C), 68.5 (4""-C), 67.0 (5""-C)
   3""-*O*-β-D-xylopyranosyl moiety; δ105.6 (1""'-C), 72.2 (2""'-C), 77.8 (3""'-C), 68.1 (4""'-C), 66.3 (5""'-C), 170.8 (3""'-C=O), 21.9 (3""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 6 shows ¹³C-NMR spectrum of the fraction 9 derived from the water extract of soybean embryo. In Figure 6, the axis of abscissas is chemical shift, and the axis of ordinates is intensity of signal.
   The fraction 9 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 9 derived from the water extract of soybean embryo is 3*-O*-[β-D-glucopyranosyl(1→2)-β-D-galactopyranosyl-(1→2)-β-D-glucuronopyranosyl]-22-*O*-[3-*O*-acetyl-β-D-xylopyranosyl(1→3)-α-L-arabinopyranosyl]soyasapogenol A (molecular weight: 1280). The compound is a compound b in Table 1 mentioned above.
(2) The enhancing activity for NGF production of the compound b was assayed in the same manner as in item (2) of Example 2. As a result, it was clarified that the compound b has an enhancing activity for NGF production. The results are shown in Table 2. The amount of NGF production of the control was 0.739 ng/mL.

### Example 4 Enhancing Activity for NGF Production of 3-O-[β-D-glucopyranosyl(1→2)-β-D-galactopyranosyl-(1→2)-β-D-glucuronopyranosyl]-22-O-[4-O-acetyl-β-D-xylopyranosyl(1→3)-α-L-arabinopyranosyl]soyasapogenol A

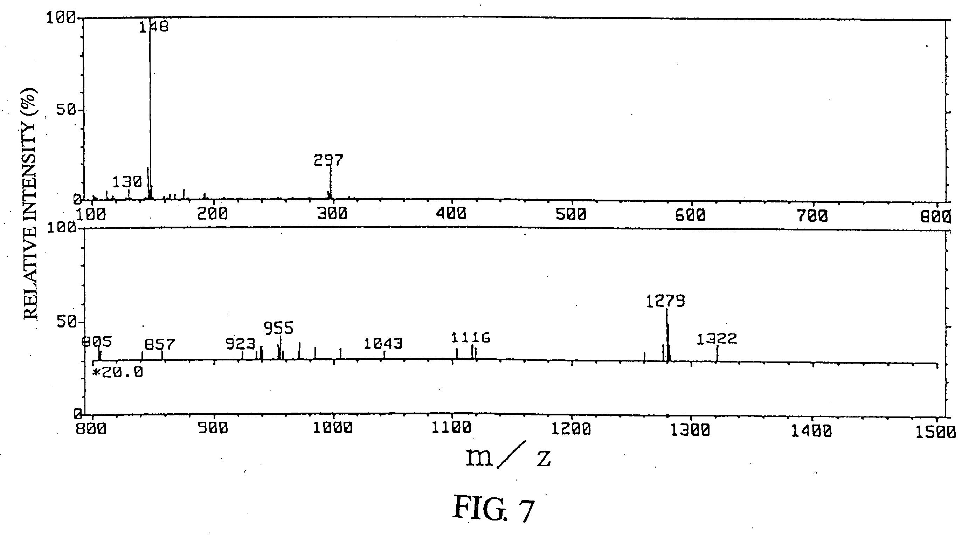

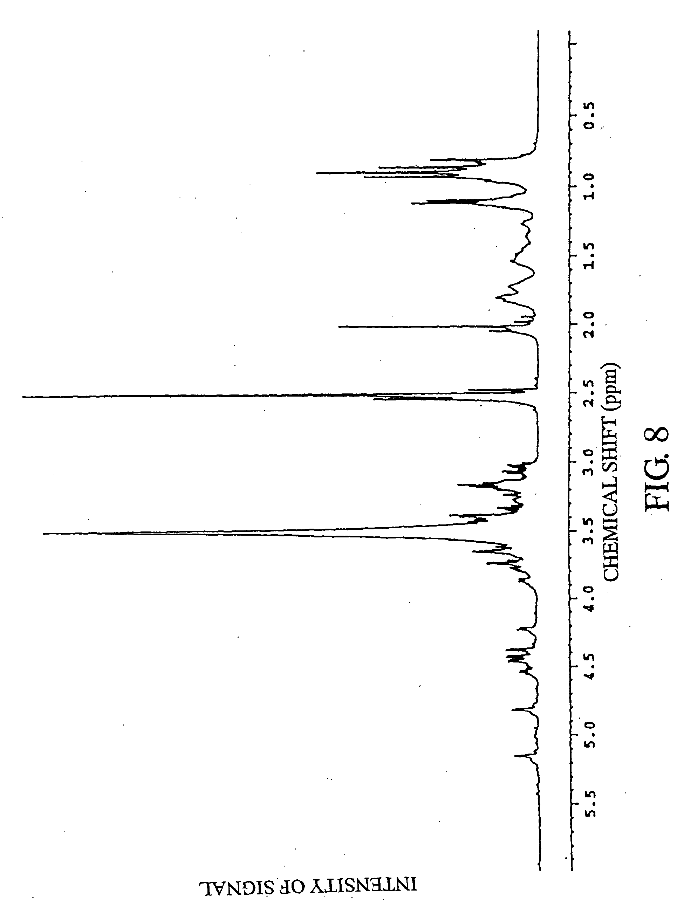

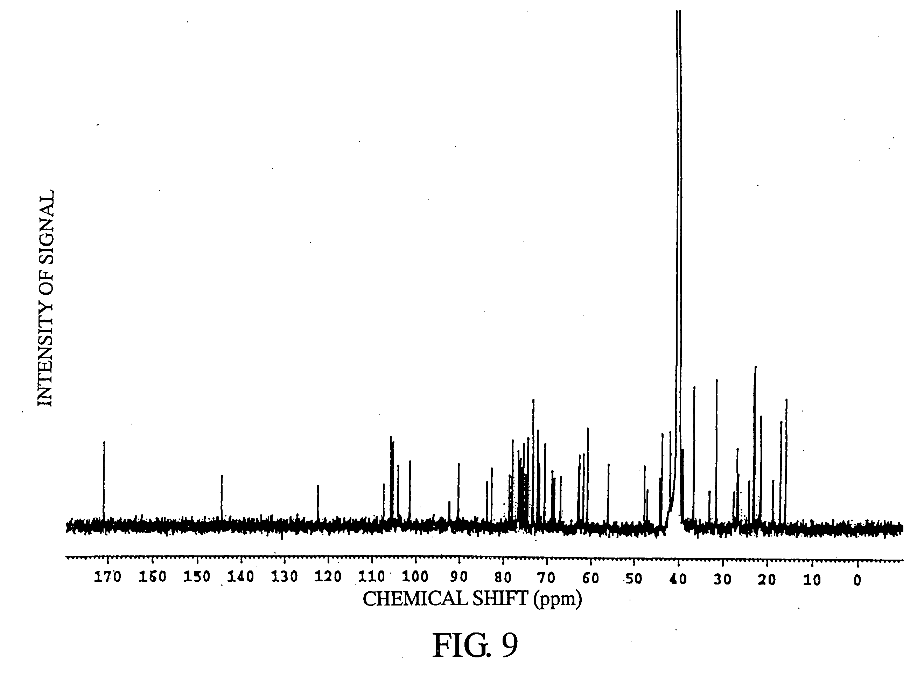

(1) The mass spectrum of the fraction 10 derived from the water extract of soybean embryo (fraction including a peak detected at a retention time of 35.6 minutes) fractionated in item (6) of Example 1 was measured in the same manner as in item (1) of Example 2. As the matrix, triethanolamine was used. According to mass spectrometry, a peak of m/z 1279 (M-H)⁻ was detected. Figure 7 shows the mass spectrum of the fraction 10 derived from the water extract of soybean embryo. In Figure 7, the axis of abscissas is m/z value, and the axis of ordinates is relative intensity.
   The NMR spectrum of the fraction 10 derived from the water extract of soybean embryo was measured in the same manner as in item (1) of Example 2. The signals of NMR are shown below.
   ¹H-NMR:
   sapogenol moiety; δ3.26 (1H, m, 3-H), 5.15 (1H, br-s, 12-H), 3.39 (1H, m, 21-H),
   3.23 (1H, br-s, 22-H), 3.15 (1H, m, 24-H), 3.86 (1H, m, 24-H)
   3-*O*-β-D-glucronopyranosyl moiety; δ4.46 (1H, d, J=7.2 Hz, 1'-H),
   3.43 (1H, m, 2'-H), 3.56 (1H, m, 3'-H), 3.33 (1H, m, 4'-H), 3.65 (1H, m, 5'-H)
   2'-*O*-β-D-galactopyranosyl moiety; δ4.81 (1H, d, J=7.2 Hz, 1"-H),
   3.51 (1H, m, 2"-H), 3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.38 (1H, d, J=7.8 Hz, 1"'-H),
   3.02 (1H, m, 2"'-H), 3.16 (1H, m, 3"'-H), 3.08 (1H, m, 4"'-H),
   3.15 (1H, m, 5"'-H), 3.47 (1H, m, 6"'-H), 3.73 (1H, m, 6"'-H)
   22-*O*-α-L-arabinopyranosyl moiety; δ4.22 (1H, d, J=7.2 Hz, 1""-H),
   3.57 (1H, m, 2""-H), 3.46 (1H, m, 3""-H), 3.74 (1H, m, 4""-H),
   3.39 (1H, m, 5""-H), 3.62 (1H, m, 5""-H)
   3""-*O*-β-D-xylopyranosyl moiety; δ4.43 (1H, d, J=7.2 Hz, 1""'-H);
   3.39 (1H, m, 2""'-H), 3.18 (1H, m, 3""'-H), 4.53 (1H, m, 4""'-H),
   3.17 (1H, m, 5""'-H), 3.78 (1H, m, 5""'-H), 2.01 (3H, s, 4""'-CH₃)
   Here, in ¹H-NMR, the sample was dissolved in deuterated dimethyl sulfoxide, and the chemical shift of deuterated dimethyl sulfoxide was expressed as 2.51 ppm. Figure 8 shows ¹H-NMR spectrum of the fraction 10 derived from the water extract of soybean embryo. In Figure 8, the axis of abscissas is chemical shift, and the axis of ordinates is intensity of signal.
   ¹³C-NMR:
   sapogenol moiety; δ 90.3 (3-C), 122.5 (12-C), 144.4 (13-C), 75.0 (21-C), 92.4 (22-C), 63.0 (24-C)
   3-*O*-β-D-glucronopyranosyl moiety; δ104.1 (1'-C), 78.8 (2'-C), 76.5 (3'-C), 71.9 (4'-C), 76.2 (5'-C), 171.0 (6'-C)
   2'-*O*-β-D-galactopyranosyl moiety; δ101.5 (1"-C), 82.7 (2"-C), 73.3 (3"-C), 69.0 (4"-C), 75.7 (5"-C), 60.9 (6"-C)
   2"-*O*-β-D-glucopyranosyl moiety; δ105.3 (1"'-C), 75.5 (2"'-C), 76.8 (3"'-C), 70.6 (4"'-C), 78.1 (5"'-C), 61.8 (6"'-C)
   22-*O*-α-L-arabinopyranosyl moiety; δ107.5 (1""-C), 71.8 (2""-C), 83.9 (3""-C), 68.4 (4""-C), 67.0 (5""-C)
   3""-*O*-β-D-xylopyranosyl moiety; δ105.9 (1""'-C), 73.3 (2""'-C), 74.5 (3""'-C), 72.3 (4""'-C), 62.8 (5""'-C), 171.0 (4""'-C=O), 21.7 (4""'-CH₃) dimethyl
   Here, in ¹³C-NMR, the sample was dissolved in deuterated dimethyl sulfoxide, and the chemical shift of deuterated dimethyl sulfoxide was expressed as 40.2 ppm. Figure 9 shows ¹³C-NMR spectrum of the fraction 10 derived from the water extract of soybean embryo. In Figure 9, the axis of abscissas is chemical shift, and the axis of ordinates is intensity of signal.
   The fraction 10 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 10 derived from the water extract of soybean embryo is 3-*O*-[β-D-glucopyranosyl(1→2)-β-D-galactopyranosyl-(1→2)-β-D-glucuronopyranosyl]-22-*O*-[4-*O*-acetyl-β-D-xylopyranosyl(1→3)-α-L-arabinopyranosyl]soyasapogenol A (molecular weight: 1280). The compound is a compound c in Table 1 mentioned above.
(2) The enhancing activity for NGF production of the compound c was assayed in the same manner as in item (2) of Example 2. As a result, it was clarified that the compound c has an enhancing activity for NGF production. The results are shown in Table 2. The amount of NGF production of the control was 0.739 ng/mL.

### Example 5 Enhancing Activity for NGF Production of 3-O-[β-D-glucopyranosyl(1→2)-β-D-galactopyranosyl-(1→2)-β-D-glucuronopyranosyl]-22-O-[2,3-di-O-acetyl-β-D-xylopyranosyl(1→3)-α-L-arabinopyranosyl]soyasapogenol A

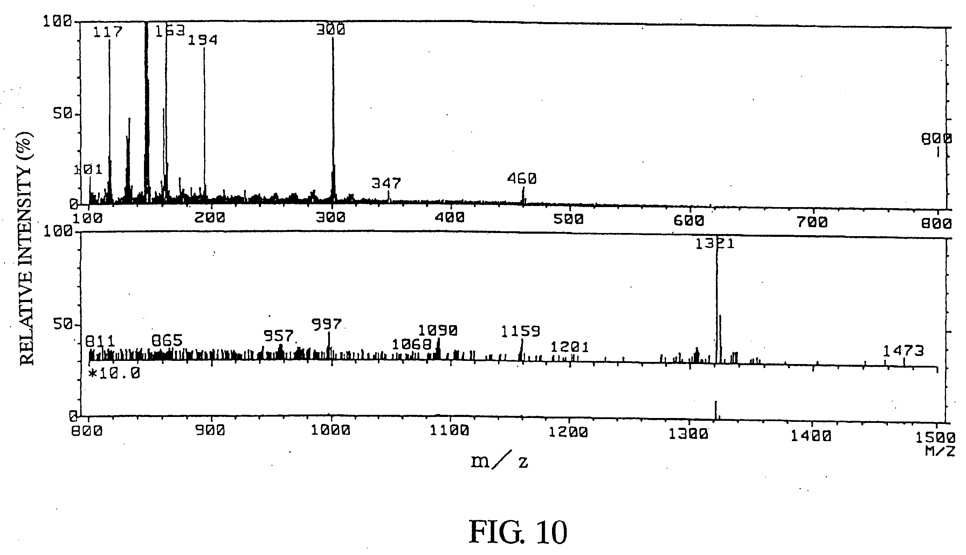

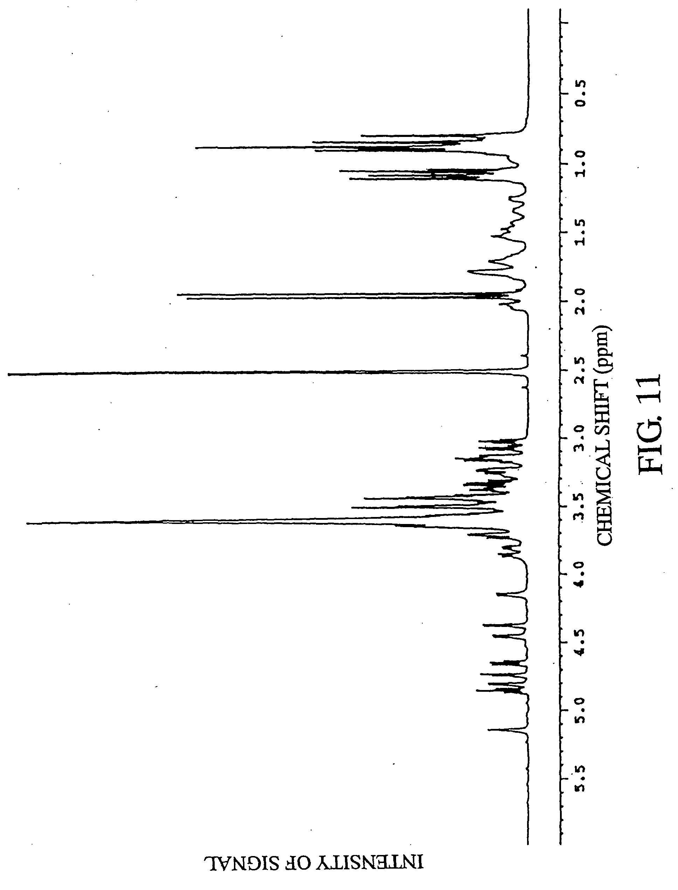

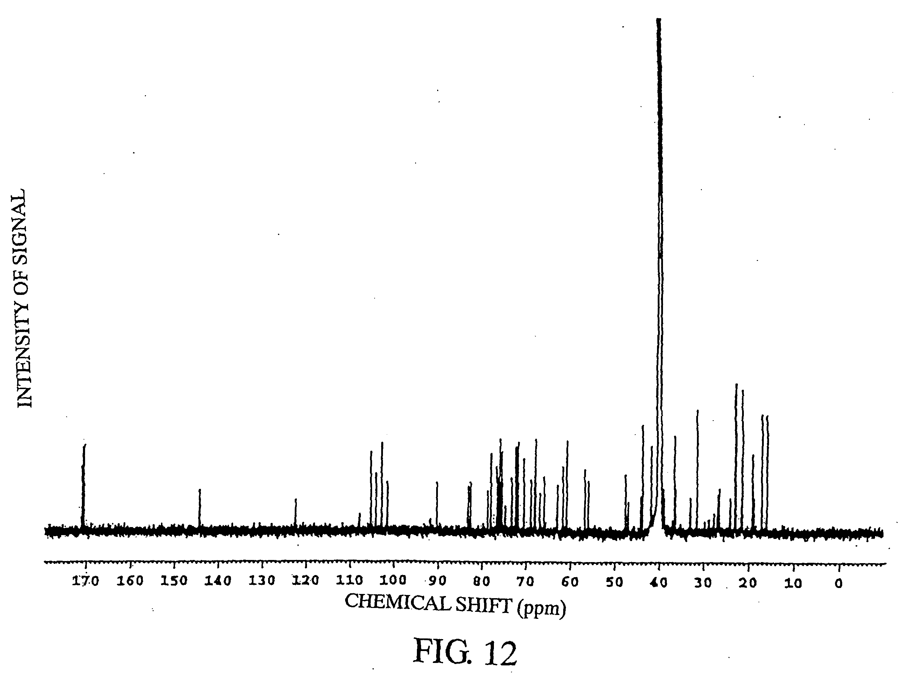

(1) The mass spectrum of the fraction 12 derived from the water extract of soybean embryo (fraction including a peak detected at a retention time of 38.2 minutes) fractionated in item (6) of Example 1 was measured in the same manner as in item (1) of Example 2. As the matrix, m-nitrobenzyl alcohol was used. According to mass spectrometry, a peak of m/z 1321(M-H)⁻ was detected. Figure 10 shows the mass spectrum of the fraction 12 derived from the water extract of soybean embryo. In Figure 10, the axis of abscissas is m/z value, and the axis of ordinates is relative intensity.
   The NMR spectrum of the fraction 12 derived from the water extract of soybean embryo was measured in the same manner as in item (1) of Example 2. The signals of NMR are shown below.
   ¹H-NMR:
   sapogenol moiety; δ3.26(1H, m, 3-H), 5.14(1H, br-s, 12-H),
   3.43 (1H, m, 21-H), 3.23 (1H, br-s, 22-H), 3.15 (1H, m, 24-H),
   3.86 (1H, m, 24-H)
   3-*O*-β-D-glucronopyranosyl moiety; δ4.46(1H, d, J=7.8 Hz, 1'-H),
   3.44 (1H, m, 2'-H), 3.56 (1H, m, 3'-H), 3.33 (1H, m, 4'-H), 3.65 (1H, m, 5'-H)
   2'-*O*-β-D-galactopyranosyl moiety; δ4.80 (1H, d, J=6.6 Hz, 1"-H),
   3.50 (1H, m, 2"-H), 3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.37 (1H, d, J=7.8 Hz, 1"'-H),
   3.03 (1H, m, 2"'-H), 3.17 (1H, m, 3"'-H), 3.08 (1H, m, 4"'-H),
   3.14 (1H, m, 5"'-H), 3.47 (1H, m, 6"'-H), 3.73 (1H, m, 6"'-H)
   22-*O*-α-L-arabinopyranosyl moiety; δ4.15 (1H, d, J=6.6 Hz, 1""-H),
   3.42 (1H, m, 2""-H), 3.45 (1H, m, 3""-H), 3.71 (1H, m, 4""-H),
   3.35 (1H, m, 5""-H), 3.62 (1H, m, 5""-H)
   3""-*O*-β-D-xylopyranosyl moiety; δ4.74 (1H, d, J=7.2 Hz, 1""'-H),
   4.65 (1H, m, 2""'-H), 4.83 (1H, t, J=7.2 Hz, 3""'-H), 3.60 (1H, m, 4""'-H),
   3.82 (1H, m, 5""'-H), 3.24 (1H, m, 5""'-H), 1.95 (3H, s, 2""'-CH₃),
   1.98 (3H, s, 3""'-CH₃)
   Here, in ¹H-NMR, the sample was dissolved in deuterated dimethyl sulfoxide, and the chemical shift of the residual proton of deuterated dimethyl sulfoxide was expressed as 2.51 ppm. Figure 11 shows ¹H-NMR spectrum of the fraction 12 derived from the water extract of soybean embryo. In Figure 11, the axis of abscissas is chemical shift, and the axis of ordinates is intensity of signal.
   ¹³C-NMR:
   sapogenol moiety; δ 90.3 (3-C), 122.5 (12-C), 144.4 (13-C), 74.8 (21-C), 91.8 (22-C), 63.0 (24-C)
   3-*O*-β-D-glucronopyranosyl moiety; δ104.1 (1'-C), 79.0 (2'-C), 76.6 (3'-C), 71.9 (4'-C), 76.1 (5'-C), 171.0 (6'-C)
   2'-*O*-β-D-galactopyranosyl moiety; δ101.5 (1"-C), 82.7 (2"-C), 73.3 (3"-C), 69.0 (4"-C), 75.8 (5"-C), 60.9 (6"-C)
   2"-*O*-β-D-glucopyranosyl moiety; δ105.3 (1"'-C), 75.5 (2"'-C), 76.7 (3"'-C), 70.6 (4"'-C), 78.1 (5"'-C), 61.8 (6"'-C)
   22-*O*-α-L-arabinopyranosyl moiety; δ107.9 (1""'-C), 72.0(2""'-C), 83.5 (3""'-C), 68.4 (4""'-C), 67.0 (5""'-C)
   3""-*O*-β-D-xylopyranosyl moiety; δ102.8 (1""'-C), 72.4 (2""'-C), 76.0 (3""'-C), 68.0 (4""'-C), 66.0 (5""'-C), 170.4 (2"""-C=O), 21.5 (2""'-CH3),
   170.7 (3""'-C=O), 21.5 (3""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 12 shows ¹³C-NMR spectrum of the fraction 12 derived from the water extract of soybean embryo. In Figure 12, the axis of abscissas is chemical shift, and the axis of ordinates is intensity of signal.
   The fraction 12 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 12 derived from the water extract of soybean embryo is 3-*O*-[β-D-glucopyranosyl(1→2)-β-D-galactopyranosyl-(1→2)-β-D-glucuronopyranosyl]-22-*O*-[2,3-di-*O*-acetyl-β-D-xylopyranosyl(1→3)-α-L-arabinopyranosyl]soyasapogenol A (molecular weight: 1322). The compound is a compound d in Table 1 mentioned above.
(2) The enhancing activity for NGF production of the compound d was assayed in the same manner as in item (2) of Example 2. As a result, it was clarified that the compound d has an enhancing activity for NGF production. The results are shown in Table 2. The amount of NGF production of the control was 0.739 ng/mL.

### Example 6 Enhancing Activity for NGF Production of 3-O-[β-D-glucopyranosyl(1→2)-β-D-galactopyranosyl-(1→2)-β-D-glucuronopyranosyl]-22-O-[2,4-di-O-acetyl-β-D-xylopyranosyl(1→3)-α-L-arabinopyranosyl]soyasapogenol A

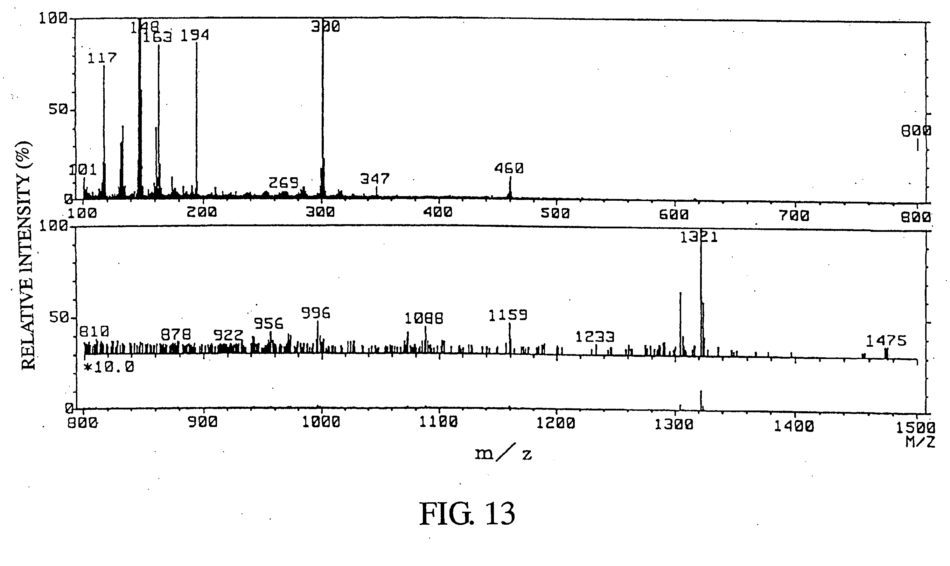

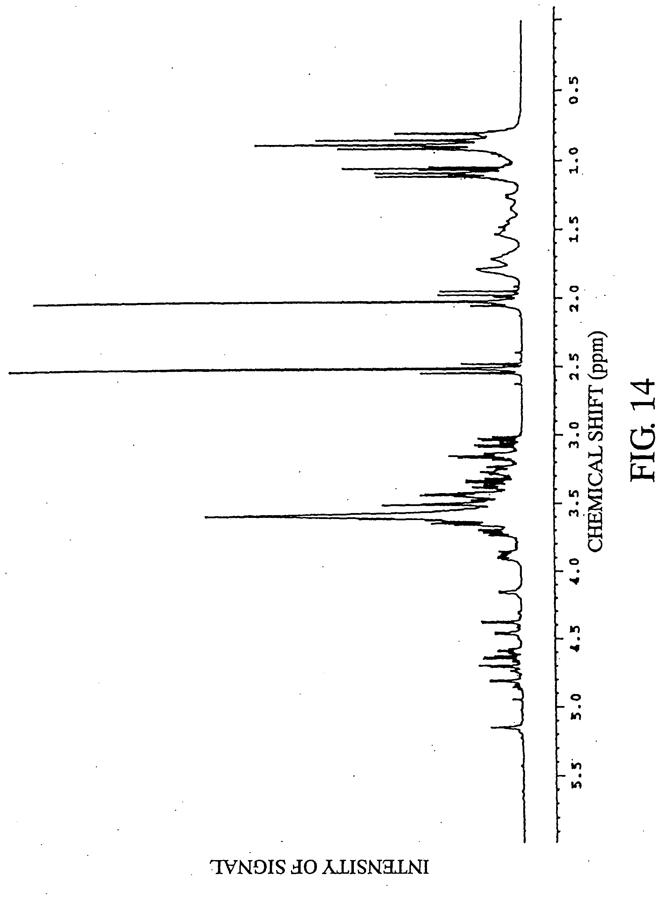

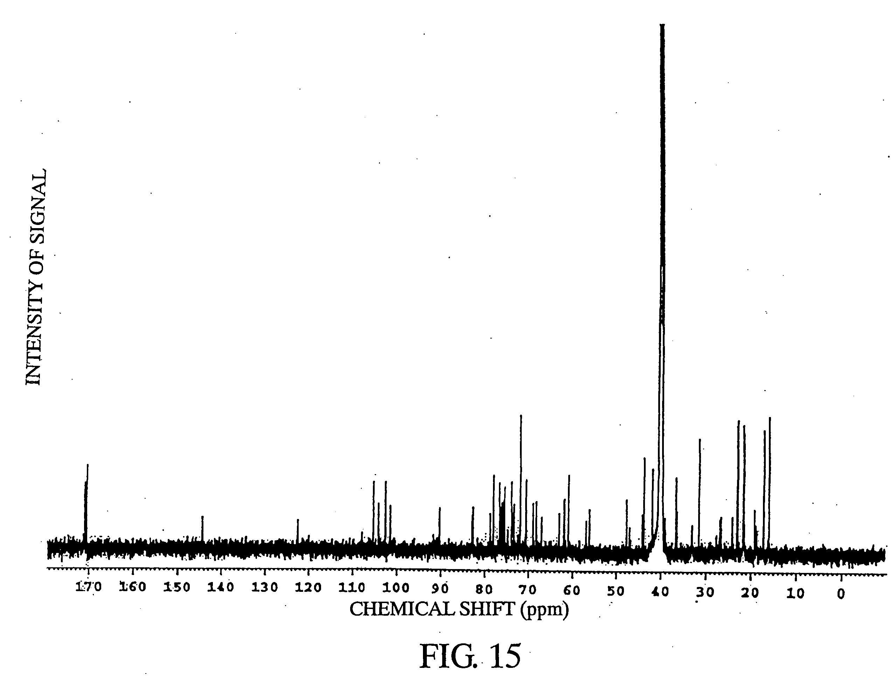

(1) The mass spectrum of the fraction 13 derived from the water extract of soybean embryo (fraction including a peak detected at a retention time of 38.9 minutes) fractionated in item (6) of Example 1 was measured in the same manner as in item (1) of Example 2. As the matrix, m-nitrobenzyl alcohol was used. According to mass spectrometry, a peak of m/z 1321(M-H)⁻ was detected. Figure 13 shows the mass spectrum of the fraction 13 derived from the water extract of soybean embryo. In Figure 13, the axis of abscissas is m/z value, and the axis of ordinates is relative intensity.
   The NMR spectrum of the fraction 13 derived from the water extract of soybean embryo was measured in the same manner as in item (1) of Example 2. The signals of NMR are shown below.
   ¹H-NMR:
   sapogenol moiety; δ3.27 (1H, m, 3-H), 5.14 (1H, br-s, 12-H), 3.45 (1H, m, 21-H),
   3.22 (1H, br-s, 22-H), 3.12 (1H, m, 24-H), 3.85 (1H, m, 24-H)
   3-*O*-β-D-glucronopyranosyl moiety; δ4.46 (1H, d, J=7.2 Hz, 1'-H),
   3.43 (1H, m, 2'-H), 3.57 (1H, m, 3'-H), 3.33 (1H, m, 4'-H), 3.64 (1H, m, 5'-H)
   2'-*O*-β-D-galactopyranosyl moiety; δ4.81 (1H, d, J=6.6 Hz, 1"-H),
   3.51 (1H, m, 2"-H), 3.51 (1H, m, 3"-H), 3.64 (1H, m, 4"-H),
   3.38 (1H; m, 5"-H), 3.50 (2H, m, 6"-H)
   2"-*O*-β-D-glucopyranosyl moiety; δ4.38 (1H, d, J=7.8 Hz, 1"'-H),
   3.02 (1H, m, 2"'-H), 3.17 (1H, m, 3"'-H), 3.08 (1H, m, 4"'-H),
   3.14 (1H, m, 5"'-H), 3.47 (1H, m, 6"'-H), 3.72 (1H, m, 6"'-H)
   22-*O*-α-L-arabinopyranosyl moiety; δ4.15 (1H, d, 1""-H), 3.44 (1H, m, 2""-H),
   3.43 (1H, m, 3""-H), 3.69 (1H, m, 4""-H), 3.35 (1H, m, 5""-H),
   3.60 (1H, m, 5""-H)
   3""-*O*-β-D-xylopyranosyl moiety; δ4.70 (1H, d, J=7.2 Hz, 1""'-H),
   4.62 (1H, m, 2""'-H), 3.60 (1H, m, 3""'-H), 4.59 (1H, m, 4""'-H),
   3.27 (1H, m, 5""'-H), 3.90 (1H, m, 5""'-H), 2.02 (3H, s, 2""'-CH₃),
   2.02 (3H, s, 4""'-CH₃)
   Here, in ¹H-NMR, the sample was dissolved in deuterated dimethyl sulfoxide, and the chemical shift of the residual proton of deuterated dimethyl sulfoxide was expressed as 2.51 ppm. Figure 14 shows ¹H-NMR spectrum of the fraction 13 derived from the water extract of soybean embryo. In Figure 14, the axis of abscissas is chemical shift, and the axis of ordinates is intensity of signal.
   ¹³C-NMR:
   sapogenol moiety; δ90.3 (3-C), 122.5 (12-C), 144.4 (13-C), 74.8 (21-C), 91.8 (22-C), 63.0 (24-C)
   3-*O*-β-D-glucronopyranosyl moiety; δ104.1 (1'-C), 78.7 (2'-C), 76.6 (3'-C), 71.9 (4'-C), 76.1 (5'-C), 171.0 (6'-C)
   2'-*O*-β-D-galactopyranosyl moiety; δ101.5 (1"-C), 82.7 (2"-C), 73.3 (3"-C), 69.0 (4"-C), 75.5 (5"-C), 60.9 (6"-C)
   2"-*O*-β-D-glucopyranosyl moiety; δ105.3 (1"'-C), 75.5 (2"'-C), 76.7 (3"'-C), 70.6 (4"'-C), 78.1 (5"'-C), 61.8 (6"'-C)
   22-*O*-α-L-arabinopyranosyl moiety; δ108.0 (1""-C), 71.9 (2""-C), 83.0 (3""-C), 68.2 (4""-C), 67.0 (5""-C)
   3""-*O*-β-D-xylopyranosyl moiety; δ102.6 (1""'-C), 73.7 (2""'-C), 70.6 (3""'-C), 71.6 (4""'-C), 62.0 (5""'-C),
   170.4, 170.9 (2""' and 4""'-C=O), 21.6, 21.8 (2""' and 4""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 15 shows ¹³C-NMR spectrum of the fraction 13 derived from the water extract of soybean embryo. In Figure 15, the axis of abscissas is chemical shift, and the axis of ordinates is intensity of signal.
   The fraction 13 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 13 derived from the water extract of soybean embryo is 3-*O*-[β-D-glucopyranosyl(1→2)-β-D-galactopyranosyl-(1→2)-β-D-glucuronopyranosyl]-22-*O*-[2,4-di-*O*-acetyl-β-D-xylopyranosyl(1→3)-α-L-arabinopyranosyl]soyasapogenol A (molecular weight: 1322). The compound is a compound e in Table 1 mentioned above.
(2) The enhancing activity for NGF production of the compound e was assayed in the same manner as in item (2) of Example 2. As a result, it was clarified that the compound e has an enhancing activity for NGF production. The results are shown in Table 2. The amount of NGF production of the control was 0.578 ng/mL.

### Example 7 Enhancing Activity for NGF Production of 3-O-[β-D-glucopyranosyl(1→2)-β-D-galactopyranosyl-(1→2)-β-D-glucuronopyranosyl]-22-O-[3,4-di-O-acetyl-β-D-xylopyranosyl(1→3)-α-L-arabinopyranosyl]soyasapogenol A

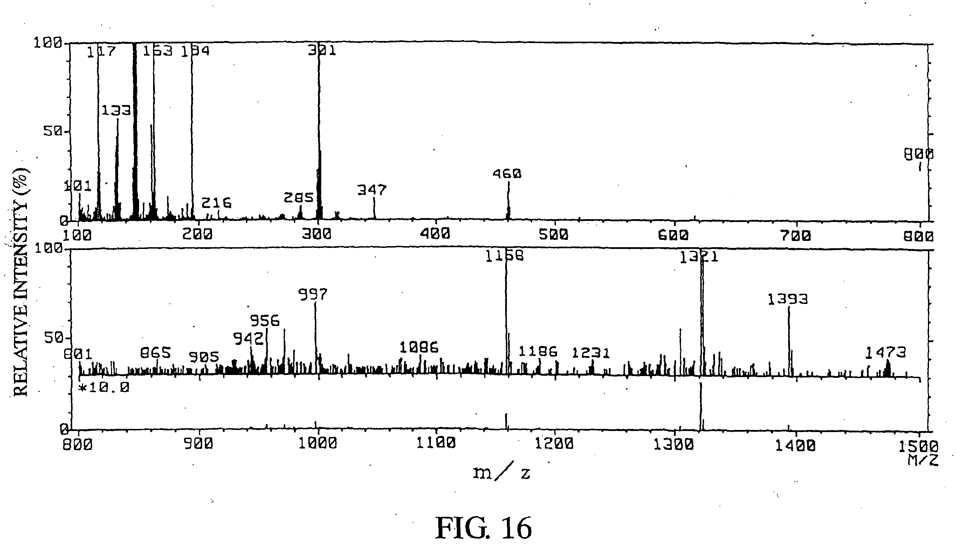

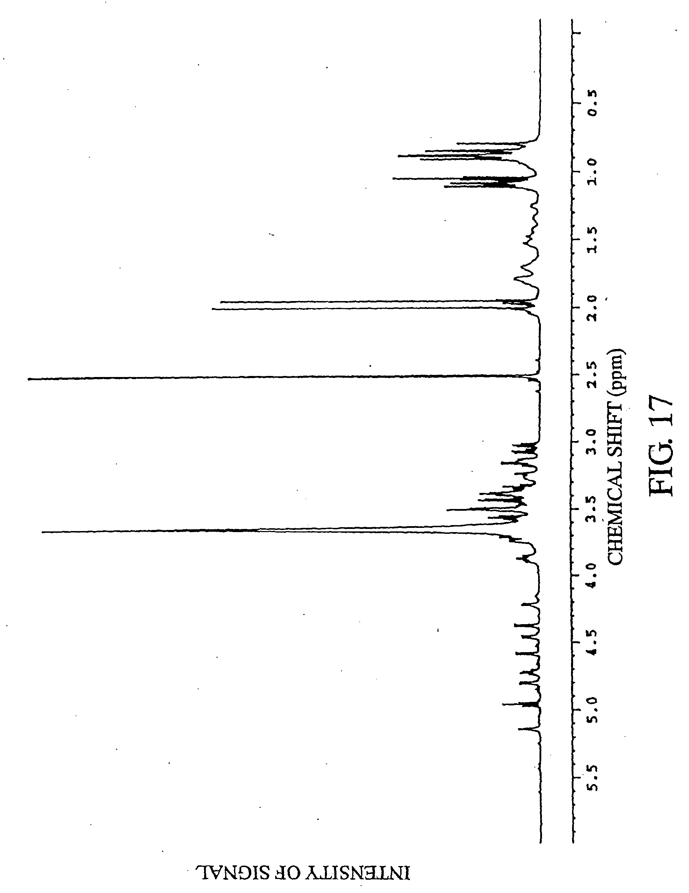

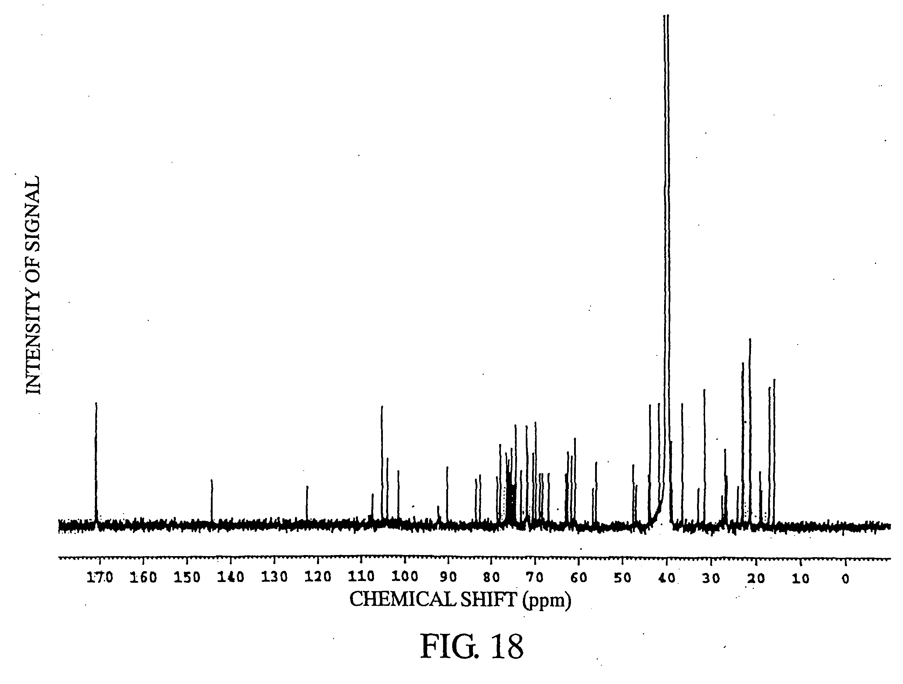

(1) The mass spectrum of the fraction 15 derived from the water extract of soybean embryo (fraction including a peak detected at a retention time of 40.3 minutes) fractionated in item (6) of Example 1 was measured in the same manner as in item (1) of Example 2. As the matrix, m-nitrobenzyl alcohol was used. According to mass spectrometry, a peak of m/z 1321(M-H)⁻ was detected. Figure 16 shows the mass spectrum of the fraction 15 derived from the water extract of soybean embryo. In Figure 16, the axis of abscissas is m/z value, and the axis of ordinates is relative intensity.
   The NMR spectrum of the fraction 15 derived from the water extract of soybean embryo was measured in the same manner as in item (1) of Example 2. The signals of NMR are shown below.
   ¹H-NMR:
   sapogenol moiety; δ3.27 (1H, m, 3-H), 5.14 (1H, br-s, 12-H), 3.39 (1H, m, 21-H),
   3.24 (1H, br-s, 22-H), 3.14 (1H, m, 24-H), 3.85 (1H, m, 24-H)
   3-*O*-β-D-glucronopyranosyl moiety; δ4.45 (1H, d, J=7.8 Hz, 1'-H),
   3.43 (1H, m, 2'-H), 3.56 (1H, t, J=8.9 Hz, 3'-H), 3.34 (1H, t, J=8.9 Hz, 4'-H), 3.66 (1H, m, 5'-H)
   2'-*O*-β-D-galactopyranosyl moiety; δ4.80 (1H, d, J=6.6 Hz, 1"-H),
   3.51 (1H, m, 2"-H), 3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.37 (1H, d, J=7.8 Hz, 1"'-H),
   3.02 (1H, dd, J=7.8, 9.0 Hz, 2"'-H), 3.17 (1H, t, J=9.0 Hz, 3"'-H),
   3.08 (1H, t, J=9.0 Hz, 4"'-H), 3.15 (1H, m, 5"'-H), 3.47 (1H, m, 6"'-H), 3.72 (1H, m, 6"'-H)
   22-*O*-α-L-arabinopyranosyl moiety; δ4.22 (1H, d, J=4.8 Hz, 1""-H),
   3.46 (1H, m, 2""-H), 3.42 (1H, m, 3""-H), 3.74 (1H, m, 4""-H),
   3.39 (1H, m, 5""-H), 3.63 (1H, m, 5""-H)
   3""-*O*-β-D-xylopyranosyl moiety; δ4.58 (1H, d, J=7.2 Hz, 1""'-H),
   3.38 (1H, m, 2""'-H), 4.97 (1H, t, J=9.3 Hz, 3""'-H), 4.72 (1H, m, 4""'-H),
   3.88 (1H, m, 5""'-H), 3.36 (1H, m, 5""'-H), 2.01, 1.96 (3H, s, 3""' and 4""'-CH₃)
   Here, in ¹H-NMR, the sample was dissolved in deuterated dimethyl sulfoxide, and the chemical shift of the residual proton of deuterated dimethyl sulfoxide was expressed as 2.51 ppm. Figure 17 shows ¹H-NMR spectrum of the fraction 15 derived from the water extract of soybean embryo. In Figure 17, the axis of abscissas is chemical shift, and the axis of ordinates is intensity of signal.
   ¹³C-NMR: δ
   sapogenol moiety; δ90.3 (3-C), 122.5 (12-C), 144.4 (13-C), 75.0 (21-C), 92.3 (22-C), 62.6 (24-C)
   3-*O*-β-D-glucronopyranosyl moiety; δ104.1 (1'-C), 78.8 (2'-C), 76.5 (3'-C), 72.0 (4'-C), 76.1 (5'-C), 171.0 (6'-C)
   2'-*O*-β-D-galactopyranosyl moiety; δ101.5 (1"-C), 82.7(2"-C), 73.4 (3"-C), 69.0 (4"-C), 75.5(5"-C), 60.9(6"-C)
   2"-*O*-β-D-glucopyranosyl moiety; δ105.3(1"'-C), 75.5(2"'-C), 76.5 (3"'-C), 70.6 (4"'-C), 78.1 (5"'-C), 61.8(6"'-C)
   22-*O*-α-L-arabinopyranosyl moiety; δ107.4 (1""-C), 72.0 (2""-C), 83.7 (3""-C), 68.3 (4""-C), 67.0(5""-C)
   3""-*O*-β-D-xylopyranosyl moiety; δ105.3 (1""'-C), 72.0 (2""'-C), 74.2 (3""'-C), 69.9 (4""'-C), 62.6 (5""'-C), 171.0, 170.7 (3""' and 4""'-C=O),
   21.3, 21.5 (3""' and 4""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 18 shows ¹³C-NMR spectrum of the fraction 15 derived from the water extract of soybean embryo. In Figure 18, the axis of abscissas is chemical shift, and the axis of ordinates is intensity of signal.
   The fraction 15 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 15 derived from the water extract of soybean embryo is 3-*O*-[β-D-glucopyranosyl(1→2)-β-D-galactopyranosyl-(1→2)-β-D-glucuronopyranosyl]-22-*O*-[3,4-di-*O*-acetyl-β-D-xylopyranosyl(1→3)-α-L-arabinopyranosyl]soyasapogenol A (molecular weight: 1322). The compound is a compound f in Table 1 mentioned above.
(2) The enhancing activity for NGF production of the compound f was assayed in the same manner as in item (2) of Example 2. As a result, it was clarified that the compound f has an enhancing activity for NGF production. The results are shown in Table 2. The amount of NGF production of the control was 0.578 ng/mL.

### Example 8 Enhancing Activity for NGF Production of 3-O-[β-D-glucopyranosyl(1→2)-β-D-galactopyranosyl-(1→2)-β-D-glucuronopyranosyl]-22-O-[3,4,6-tri-O-acetyl-β-D-glucopyranosyl(1→3)-α-L-arabinopyranosyl]soyasapogenol A

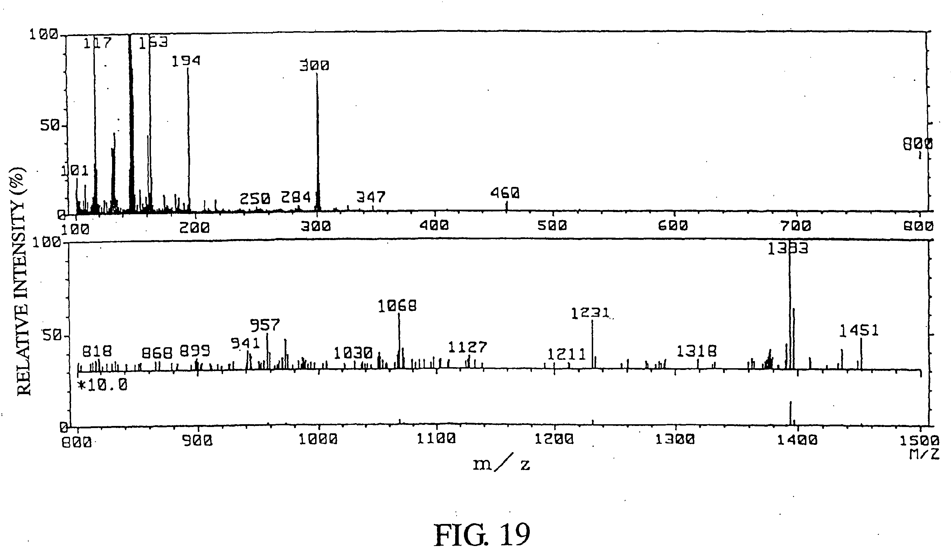

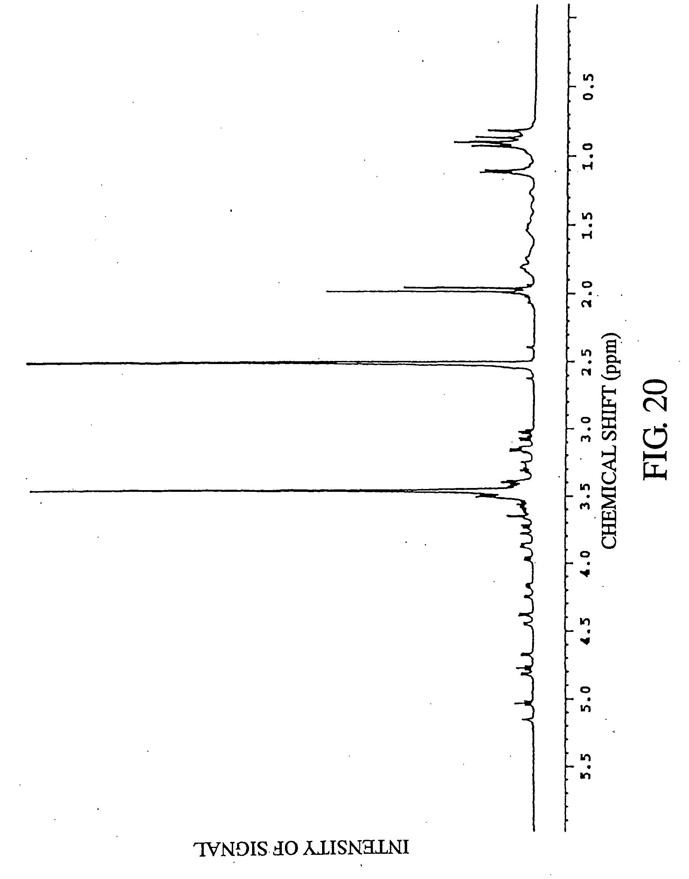

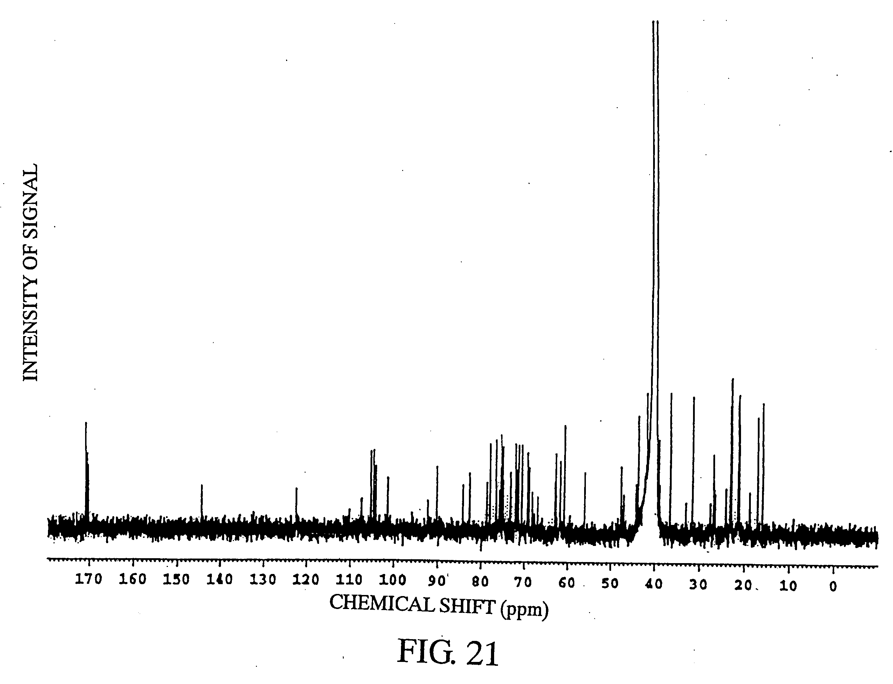

(1) The mass spectrum of the fraction 16 derived from the water extract of soybean embryo (fraction including a peak detected at a retention time of 41.0 minutes) fractionated in item (6) of Example 1 was measured in the same manner as in item (1) of Example 2. As the matrix, m-nitrobenzyl alcohol was used. According to mass spectrometry, a peak of m/z 1393 (M-H)⁻ was detected. Figure 19 shows the mass spectrum of the fraction 16 derived from the water extract of soybean embryo. In Figure 19, the axis of abscissas is m/z value, and the axis of ordinates is relative intensity.
   The NMR spectrum of the fraction 16 derived from the water extract of soybean embryo was measured in the same manner as in item (1) of Example 2. The signals of NMR are shown below.
   ¹H-NMR:
   sapogenol moiety; δ3.28 (1H, m, 3-H), 5.15 (1H, br-s, 12-H), 3.40 (1H, m, 21-H),
   3.25 (1H, br-s, 22-H), 3.15 (1H, m, 24-H), 3.87 (1H, m, 24-H)
   3-*O*-β-D-glucronopyranosyl moiety; δ4.45 (1H, d, 1'-H), 3.44 (1H, m,2'-H),
   3.57 (1H, m, 3'-H), 3.32 (1H, m, 4'-H), 3.62 (1H, m, 5'-H)
   2'-*O*-β-D-galactopyranosyl moiety; δ4.82 (1H, d, 1"-H), 3.51 (1H, m, 2"-H),
   3.51 (1H, m, 3"-H), 3.65 (1H, m, 4"-H), 3.38 (1H, m, 5"-H), 3.50 (2H, m, 6"-H)
   2"-*O*-β-D-glucopyranosyl moiety; δ4.38 (1H, d, J=7.8 Hz, 1"'-H),
   3.02 (1H, m, 2"'-H), 3.17 (1H, m, 3"'-H), 3.09 (1H, m, 4"'-H),
   3.14 (1H, m, 5"'-H), 3.47 (1H, m, 6"'-H), 3.72 (1H, m, 6"'-H)
   22-*O*-α-L-arabinopyranosyl moiety; δ4.25 (1H, d, 1""-H), 3.59 (1H, m, 2""-H),
   3.52 (1H, m, 3""-H), 3.78 (1H, m, 4""-H), 3.42 (1H, m, 5""-H),
   3.66 (1H, m, 5""-H)
   3""-*O*-β-D-glucopyranosyl moiety; δ4.67 (1H, d, J=7.8 Hz, 1""'-H),
   3.41 (1H, dd, J=7.8, 9.3 Hz, 2""'-H), 5.03 (1H, t, J=9.3 Hz, 3""'-H),
   4.77 (1H, t, J=9.3 Hz, 4""'-H), 3.87 (1H, m, 5""'-H) 3.97 (1H, m, 6""'-H),
   4.17 (1H, m, 6""'-H), 1.96, 1.99 (3H and 6H, s, 3""',4""' and 6""'-CH₃)
   Here, in ¹H-NMR, the sample was dissolved in deuterated dimethyl sulfoxide, and the chemical shift of the residual proton of deuterated dimethyl sulfoxide was expressed as 2.51 ppm. Figure 20 shows ¹H-NMR spectrum of the fraction 16 derived from the water extract of soybean embryo. In Figure 20, the axis of abscissas is chemical shift, and the axis of ordinates is intensity of signal.
   ¹³C-NMR:
   sapogenol moiety; δ 90.3 (3-C), 122.5 (12-C), 144.4 (13-C), 75.0 (21-C), 92.4 (22-C), 63.0 (24-C)
   3-*O*-β-D-glucronopyranosyl moiety; δ104.1 (1'-C), 78.8 (2'-C), 76.6 (3'-C), 72.0 (4'-C), 76.0 (5'-C), 171.0 (6'-C)
   2'-*O*-β-D-galactopyranosyl moiety; δ101.5 (1"-C), 82.8 (2"-C), 73.2 (3"-C), 69.0 (4"-C), 75.7 (5"-C), 60.9 (6"-C)
   2"-*O*-β-D-glucopyranosyl moiety; δ105.3 (1"'-C), 75.5 (2"'-C), 76.7 (3"'-C), 70.6 (4"'-C), 78.1 (5"'-C), 61.8 (6"'-C)
   22-*O*-α-L-arabinopyranosyl moiety; δ107.4 (1""-C), 71.6 (2""- C), 84.4 (3""-C), 68.3 (4""-C), 67.0 (5""-C)
   3""-*O*-β-D-glucopyranosyl moiety; δ104.7 (1""'-C), 72.2 (2""'-C), 75.0 (3""'-C), 69.4 (4""'-C), 71.3(5""'-C), 62.8 (6""'-C), 170.3, 170.5, 171.1 (3""', 4""' and
   6""'-C=O), 21.3, 21.4, 21.5 (3""', 4""' and 6""'-CH₃)
   Here, in ¹³C-NMR, the sample was dissolved in deuterated dimethyl sulfoxide, and the chemical shift of deuterated dimethyl sulfoxide was expressed as 40.2 ppm. Figure 21 shows ¹³C-NMR spectrum of the fraction 16 derived from the water extract of soybean embryo. In Figure 21, the axis of abscissas is chemical shift, and the axis of ordinates is intensity of signal.
   The fraction 16 derived from the water extract of soybean embryo was subjected to hydrolysis in the same manner as in item (1) of Example 2, to produce a saccharide component and a non-saccharide component. The non-saccharide component was subjected to thin layer chromatography (developing solvent A). As a result, a spot having the same Rf values as in Soyasapogenol A was detected. The saccharide component was subjected to thin layer chromatography (developing solvent B). As a result, arabinose, glucose, xylose and galactose were detected.
   From the above results, it was identified that the fraction 16 derived from the water extract of soybean embryo is 3-*O*-[β-D-glucopyranosyl(1→2)-β-D-galactopyranosyl-(1→2)-β-D-glucuronopyranosyl]-22-*O*-[3,4,6-tri-*O*-acetyl-β-D-glucopyranosyl(1→3)-α-L-arabinopyranosyl]soyasapogenol A (molecular weight: 1394). The compound is a compound g in Table 1 mentioned above.
(2) The enhancing activity for NGF production of the compound g was assayed in the same manner as in item (2) of Example 2. As a result, it was clarified that the compound g has an enhancing activity for NGF production. The results are shown in Table 2. The amount of NGF production of the control was 0.739 ng/mL.

### Example 9 Enhancing Activity for NGF Production of 3-O-[β-D-glucopyranosyl(1→2)-β-D-galactopyranosyl-(1→2)-β-D-glucuronopyranosyl]-22-O-[2,3,4,6-tetra-O-acetyl-β-D-glucopyranosyl(1→ 3)-α-L-arabinopyranosyl]soyasapogenol A

(1) The mass spectrum and NMR spectrum of the fraction 18 derived from water extract of soybean embryo (fraction including a peak detected at a retention time of 42.7 minutes) fractionated in item (6) of Example 1, and thin layer chromatography of the sacchatride moiety and the non-saccharide moiety were measured in the same manner as in item (1) of Example 2. As a result, it was identified that the fraction 18 derived from the water extract of soybean embryo is 3-*O*-[β-D-glucopyranosyl(1→2)-β-D-galactopyranosyl-(1→2)-β-D-glucuronopyranosyl]-22-*O*-[2,3,4,6-tetra-*O*-acetyl-β-D-glucopyranosyl(1→3)-α-L-arabinopyranosyl]soyasapogenol A (molecular weight: 1436). The compound is a compound h in Table 1 mentioned above.
(2) The enhancing activity for NGF production of the compound h was assayed in the same manner as in item (2) of Example 2. As a result, it was clarified that the compound h has enhancing activity for NGF production. The results are shown in Table 2. Incidentally, the amount of NGF production of the control was 0.898 ng/mL.

### Example 10 Fractionation of Fraction Derived from 70% Ethanol Extract of Soybean Embryo

(1) To the precipitate 2 (5.6 kg) obtained in item (1) of Example 1 was added 8.4 liters of ethanol, and the mixture was stirred at room temperature for 1 hour. Thereafter, centrifugation was carried out at 5000 g for 10 minutes, to give a precipitate 6 and supernatant 6. Ten liters of 70% ethanol was added to the precipitate 6, and the mixture was stirred for 1 hour. Thereafter, centrifugation was carried out at 5000 g for 10 minutes, to give a precipitate 7 and supernatant 7. The supernatant 6 and the supernatant 7 were mixed and concentrated under reduced pressure, to give a concentrate of 70% ethanol extract of soybean embryo.
(2) The concentrate of 70% ethanol extract of soybean embryo obtained in item (1) of Example 10 was dissolved in 500 mL of 70% ethanol. Thereafter, centrifugation was carried out at 5000 g for 10 minutes, to give a precipitate 8 and supernatant 8. The supernatant 8 was concentrated under reduced pressure, and thereafter the concentrate was suspended in 1000 mL of distilled water. The residue after filtration was obtained as a water-insoluble fraction derived from 70% ethanol extract of soybean embryo.
(3) In a solvent composed of 2 mL of methanol and 10 mL of chloroform was dissolved 0.4 volumes of the water-insoluble fraction derived from 70% ethanol extract of soybean embryo obtained in item (2) of Example 10, and the mixture was fractionated using a silica column. The conditions therefor are given below. BW-300SP (the amount of resin: 300 mL) was used as silica gel. The elution was carried out using chloroform : methanol : acetic acid = 100 : 20 : 1 (400 mL), chloroform : methanol = 2 : 1 (400 mL) and ethanol: water = 10 : 1(200 mL), in that order, as developing solvents. Fractions were collected for every 100 mL portion, to give fractions 1 to 10. Each of the eluted fractions was concentrated under reduced pressure, to give silica column fractions 1 to 10 derived from the 70% ethanol extract.
(4) The silica column fractions 4 and 5 derived form the 70% ethanol extract derived in item (3) of Example 10 were combined and concentrated, and the concentrate was then dissolved in 12 mL of ethanol. Thereafter, the mixture was fractionated using reverse phase chromatography. The conditions therefor are given below. TSK gel ODS-80Ts (21.5 mm × 30 cm) was used as the column. The elution ratio of Solvent A (mixture prepared by mixing distilled water and acetonitrile in a volume ratio of 3 : 1) to Solvent B (mixture prepared by mixing distilled water and acetonitrile in a volume ratio of 1 : 3) was such that the ratio of Solvent B increased linearly at 40 to 70% from 0 to 25 minutes, the ratio of Solvent B increased linearly at 70 to 100% in the next 25 to 26 minutes, and the ratio of Solvent B was retained at 100% during the subsequent 5 minutes. The elution rate was 5 mL/minute, and the detection was carried out at 215 nm. Fractions 1 to 10 derived from the 70% ethanol extract of soybean embryo were fractionated using ultraviolet light absorption of the eluate as an index.

### Example 11 Enhancing, Activity for NGF Production of 3-O-[β-D-glucopyranosyl(1→2)-β-D-galactopyranosyl-(1→2)-β-D-glucuronopyranosyl]-soyasapogenol B

(1) The mass spectrum and NMR spectrum of the fraction 6 derived from the 70% ethanol extract of soybean embryo (fraction including a peak detected at a retention time of 24.8 minutes) fractionated in item (4) of Example 10 were measured in the same manner as in item (1) of Example 2. Also, the fraction 6 derived from the 70% ethanol extract of soybean embryo was heated in a mixed solution of 2 M trifluoroacetic acid : 2N HCl (1 : 1) in a gas phase at 100°C for 4 hours, to produce a saccharide component and a non-saccharide component. The non-saccharide moiety was subjected to thin layer chromatography (developing solvent A). As a result, a spot having the same Rf value as Soyasapogenol B was detected. The saccharide component was subjected to thin layer chromatography (developing solvent B). As a result, glucose and galactose were detected. Consequently, it was identified that the fraction 6 derived from the 70% ethanol extract of soybean embryo is 3-*O*-[β-D-glucopyranosyl(1→2)-β-D-galactopyranosyl-(1→2)-β-D-glucuronopyranosyl]-soyasapogenol B (molecular weight: 958). The compound is a compound **i** in Table 1 mentioned above.
(2) The enhancing activity for NGF production of the compound **i** was assayed in the same manner as in item (2) of Example 2. As a result, it was clarified that the compound **i** has enhancing activity for NGF production. The results are shown in Table 2. The amount of NGF production of the control was 0.948 ng/mL.

### Example 12 Enhancing Activity for NGF Production of 3-O-[α-L-rhamnopyranosyl(1→2)-β-D-galactopyranosyl-(1→2)-β-D-glucuronopyranosyl]-soyasapogenol B

(1) The mass spectrum and NMR spectrum of the fraction 7 derived from the 70% ethanol extract of soybean embryo (fraction including a peak detected at a retention time of 27.4 minutes) fractionated in item (4) of Example 10, and thin layer chromatography of the saccharide moiety and the non-saccharide moiety were measured in the same manner as in item (1) of Example 11. As a result, it was identified that the fraction 7 derived from the 70% ethanol extract of soybean embryo is 3-*O*-[α-L-rhamnopyranosyl(1→2)-β-D-galactopyranosyl-(1→2)-β-D-glucuronopyranosyl]-soyasapogenol B (molecular weight: 942). The compound is a compound j in Table 1 mentioned above.
(2) The enhancing activity for NGF production of the compound j was assayed in the same manner as in item (2) of Example 2. As a result, it was clarified that the compound j has enhancing activity for NGF production. The results are shown in Table 2. The amount of NGF production of the control was 0.948 ng/mL.

### Example 13 Enhancing Activity for NGF Production of 3-O-[β-D-glucopyranosyl(1→2)-β-D-galactopyranosyl-(1→2)-β-D-glucuronopyranosyl]-soyasapogenol E

(1) The mass spectrum and NMR spectrum of the fraction 8 derived from the 70% ethanol extract of soybean embryo (fraction including a peak detected at a retention time of 29.3 minutes) fractionated in item (4) of Example 10, and thin layer chromatography of the saccharide moiety and the non-saccharide moiety were measured in the same manner as in item (1) of Example 11. As a result, it was identified that the fraction 8 derived from the 70% ethanol extract of soybean embryo is 3-*O*-[β-D-glucopyranosyl(1→2)-β-D-galactopyranosyl-(1→2)-β-D-glucuronopyranosyl]-soyasapogenol E (molecular weight: 956). The compound is a compound k in Table 1 mentioned above.
(2) The enhancing activity for NGF production of the compound k was assayed in the same manner as in item (2) of Example 2. As a result, it was clarified that the compound k has enhancing activity for NGF production. The results are shown in Table 2. The amount of NGF production of the control was 0.924 ng/mL.

### Example 14 Enhancing Activity for NGF Production of 3-O-[α-L-rhamnopyranosyl(1→2)-β-D-galactopyranosyl-(1→2)-β-D-glucuronopyranosyl]-soyasapogenol E

(1) The mass spectrum and NMR spectrum of the fraction 9 derived from the 70% ethanol extract of soybean embryo (fraction including a peak detected at a retention time of 31.7 minutes) fractionated in item (4) of Example 10, and thin layer chromatography of the saccharide moiety and the non-saccharide moiety were measured in the same manner as in item (1) of Example 11. As a result, it was identified that the fraction 9 derived from the 70% ethanol extract of soybean embryo is 3-*O*-[α-L-rhamnopyranosyl(1→2)-β-D-galactopyranosyl-(1→2)-β-D-glucuronopyranosyl]-soyasapogenol E (molecular weight: 940). The compound is a compound I in Table 1 mentioned above.
(2) The enhancing activity for NGF production of the compound I was measured in the same manner as in item (2) of Example 2. As a result, it was clarified that the compound **l** has enhancing activity for NGF production. The results are shown in Table 2. The amount of NGF production of the control was 0.924 ng/mL.

### Example 15 Enhancing Activity for NGF Production of 3-O-[β-D-glucopyranosyl(1→2)-β-D-galactopyranosyl-(1→2)-β-D-glucuronopyranosyl]-22-O-[2,3,4-tri-O-acetyl-β-D-xylopyranosyl(1→3)-α-L-arabinopyranosyl]soyasapogenol A

(1) The silica column fraction 3 derived from the 70% ethanol extract of soybean embryo obtained in item (3) of Example 10 was dissolved in 5 mL of ethanol, and the mixture was fractionated with reverse phase chromatography. The conditions therefor are given below. TSK gel ODS-80Ts (21.5 mm × 30 cm) was used as the column. The elution ratio of Solvent A (mixture prepared by mixing distilled water and acetonitrile in a volume ratio of 3 : 1) to Solvent B (mixture prepared by mixing distilled water and acetonitrile in a volume ratio of 1 : 3) was such that the ratio of Solvent B was increased linearly at 40 to 45% from 0 to 15 minutes, the ratio of Solvent B was increased linearly at 45 to 100% during the next 15 to 20 minutes, and the ratio of Solvent B was retained at 100% during the subsequent 5 minutes. The elution rate was 5 mL/minute, and the detection was carried out at 215 nm. Fractions were fractionated using ultraviolet light absorption of the eluate as an index.
(2) The mass spectrum and NMR spectrum of the fraction A derived from the 70% ethanol extract of soybean embryo (fraction including a peak detected at a retention time of 19.5 minutes) obtained by fractionating the silica column fraction 3 derived from the 70% ethanol extract of soybean embryo in item (1) of Example 15, and thin layer chromatography of the saccharide moiety and the non-saccharide moiety were measured in the same manner as in item (1) of Example 11. As a result, it was identified that the fraction A derived from the 70% ethanol extract of soybean embryo is 3-*O*-[β-D-glucopyranosyl(1→2)-β-D-galactopyranosyl-(1→2)-β-D-glucuronopyranosyl]-22-*O*-[2,3,4-tri-*O*-acetyl-β-D-xylopyranosyl(1→3)-α-L-arabinopyranosyl]soyasapogenol A (molecular weight: 1364). The compound is a compound m in Table 1 mentioned above.
(3) The enhancing activity for NGF production of the compound m was assayed in the same manner as in item (2) of Example 2. As a result, it was clarified that the compound m has enhancing activity for NGF production. The results are shown in Table 2. In addition, the amount of NGF production of the control was 0.898 ng/mL.

**Table 2**

| Compounds of the Present Invention | Amount of NGF Production (%) | | | |
|---|---|---|---|---|
| | Concentration of Sample (mM) | | | |
| | 0 | 0.5 | 1.0 | 2 |
| Compound **a** | 100 | 228.3 | 231.6 | 212.6 |
| Compound **b** | 100 | 220.9 | 251.2 | 272.3 |
| Compound **c** | 100 | 255.1 | 289.2 | 323.7 |
| Compound **d** | 100 | 255.8 | 281.1 | 276.5 |
| Compound **e** | 100 | 214.0 | 233.7 | 230.1 |
| Compound **f** | 100 | 204.5 | 208.8 | 218.5 |
| Compound **g** | 100 | 295.4 | 277.2 | 247.6 |
| Compound **h** | 100 | 273.3 | 301.7 | 307.2 |
| Compound **i** | 100 | N. T. | 116.8 | 169.4 |
| Compound **j** | 100 | 138.2 | 176.4 | 212.5 |
| Compound **k** | 100 | N. T. | 115.9 | 456.3 |
| Compound **l** | 100 | N. T. | 145.9 | 531.2 |
| Compound **m** | 100 | 263.3 | 250.9 | 233.4 |
| The amounts of NGF production in the sample-added group are expressed as % when the amounts of NGF production in the sample-non-added group (control) are regarded as 100%. | | | | |
| N. T. means that no test was carried out. | | | | |

### Example 16 Enhancing Activity for NGF production of 13(18)-oleanene-3,22,24-triol

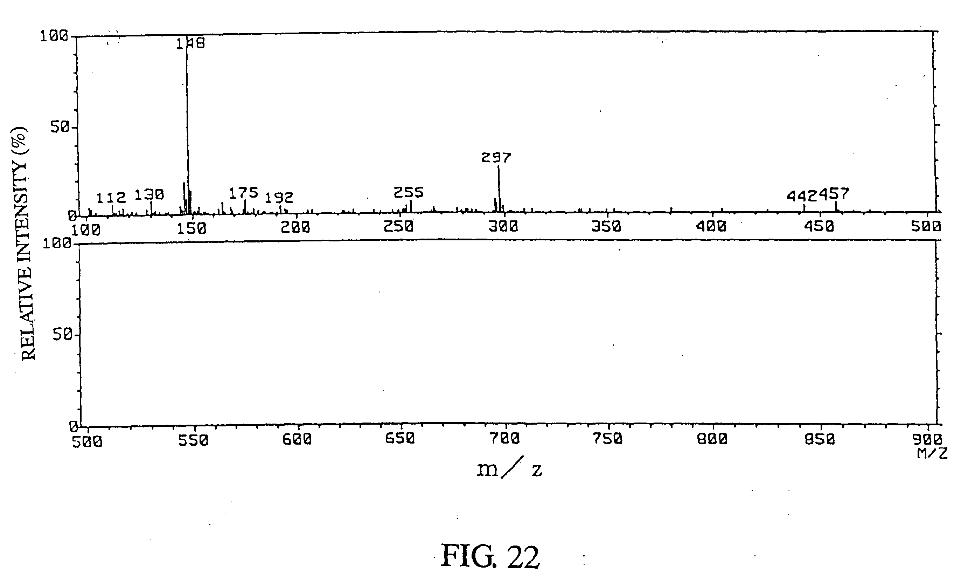

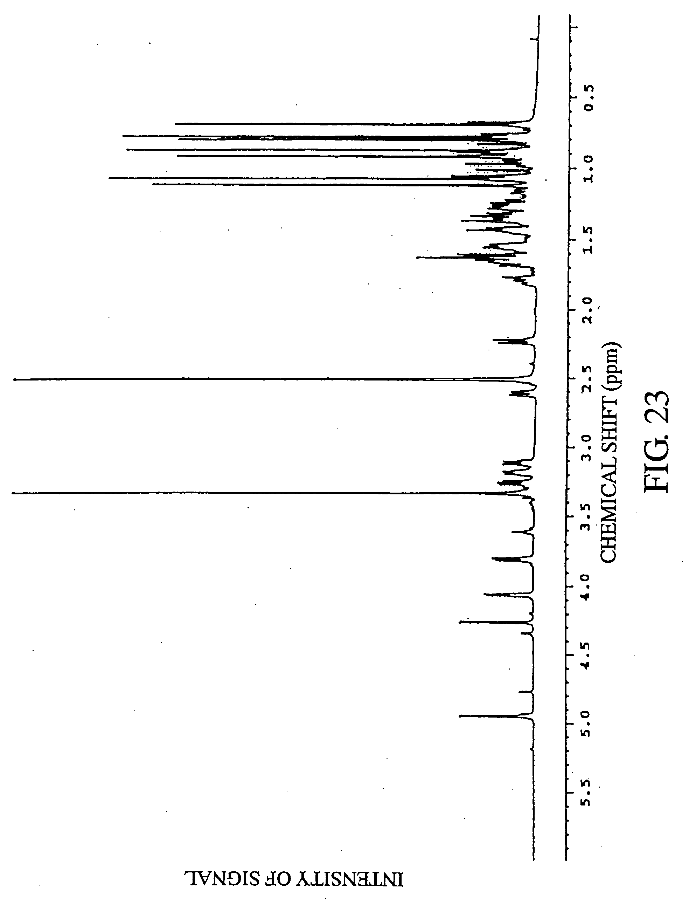

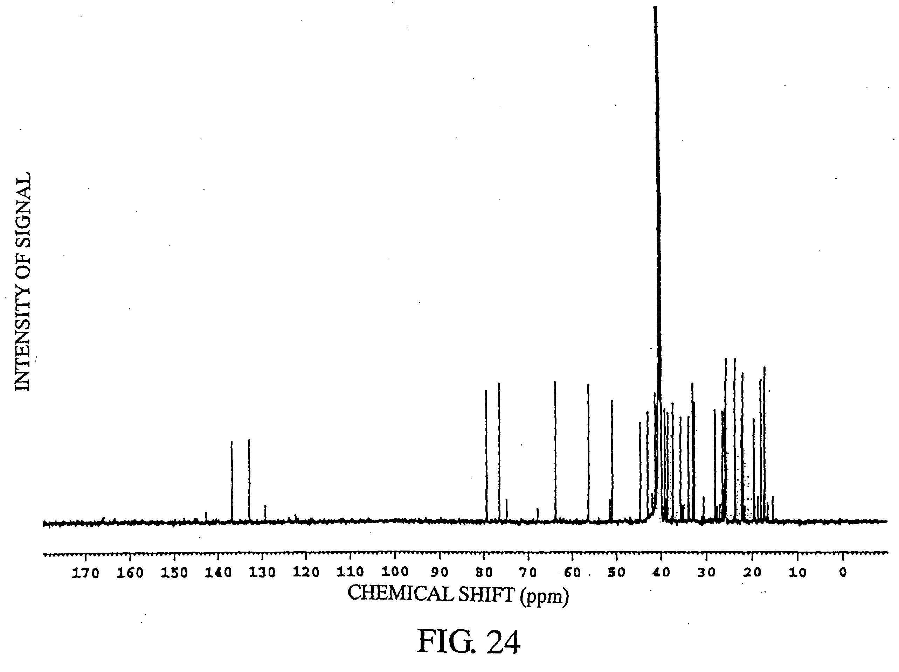

(1) Under the conditions of 50% methanol, 2 N hydrochloric acid, was heated at 80°C for 3 hours 0.04 volumes of the water-insoluble fraction derived from the 70% ethanol extract of soybean embryo obtained in item (2) of Example 10. Thereafter, the reaction solution was extracted with an equivolume of diethyl ether for 3 times in total. The ether layer obtained was concentrated under reduced pressure, to give white powder. The resulting white powder was washed with hexane, to give an acid hydrolyzate of soybean embryo.
(2) The acid hydrolyzate of soybean embryo obtained in item (1) of Example 16 was dissolved in chloroform, and the mixture was fractionated with silica chromatography. The conditions therefor are given below. BW-300SP (the amount of resin: 100 mL) was used as silica gel. The elution was carried out using chloroform : methanol = 100 : 1 as a developing solvent, to give eluted fractions of a silica column fraction 1 derived from the acid hydrolyzate of soybean embryo (550 mL), a silica column fraction 2 derived from the acid hydrolyzate of soybean embryo (300 mL), and a silica column fraction 3 derived from the acid hydrolyzate of soybean embryo (250 mL) in that order.
(3) The silica column fraction 2 derived from the acid hydrolyzate of soybean embryo obtained in item (2) of Example 16 was dissolved in 4 mL of ethanol, and the mixture was fractionated using reverse phase chromatography. The conditions are given below. TSK gel ODS-80Ts (21.5 mm × 30 cm) was used as the column. A mixture prepared by mixing water and acetonitrile in a volume ratio of 1 : 9 was used as a solvent, the elution rate was 5 mL/minute, and the detection was carried out at 215 nm. Fractions derived from the acid hydrolyzate of soybean embryo were fractionated using ultraviolet light absorption of the eluate as an index.
(4) The mass spectrum of the fraction a derived from the acid hydrolyzate of soybean embryo fractionated in item (3) of Example 16 (fraction including a peak detected at an elution time of 37.5 minutes) was measured in the same manner as in item (1) of Example 2. Triethanolamine was used as a matrix. According to mass spectrometry, a peak of m/z 457(M-H)⁻ was detected. The mass spectrum of the fraction a derived from the acid hydrolyzate of soybean embryo is shown in Figure 22. In Figure 22, the axis of abscissas is m/z value, and the axis of ordinates is relative intensity.
The NMR spectrum of the fraction **a** derived from the acid hydrolyzate of soybean embryo was measured in the same manner as in item (1) of Example 2. The signals of NMR are shown below.
¹H-NMR: δ0.70, 0.92 (3H, s, 29 and 30-CH₃), 0.77 (1H, m, 5-H), 0.79 (3H, s, 26-CH₃), 0.81 (3H, s, 25-CH₃), 0.88 (3H, s, 28-CH₃),
0.96 (1H, m, 1-H), 1.07 (1H, m, 15-H), 1.08 (3H, s, 23-CH₃), 1.12 (3H, s, 27-CH₃), 1.16 (1H, dd, J=4.8, 13.8 Hz, 11-H), 1.25 (1H, m, 16-H), 1.27(1H, m, 21-H), 1.32 (1H, m, 6-H), 1.34 (1H, m, 21-H), 1.34 (1H, m, 7-H), 1.37 (1H, m, 7-H), 1.43 (1H, m, 9-H), 1.45 (1H, m, 11-H), 1.55 (1H, m, 6-H), 1.56 (1H, m, 2-H), 1.61 (1H, m, 15-H), 1.62 (1H, m, 19-H), 1.64 (1H, m, 2-H), 1.65 (1H, m, 1-H), 1.68 (1H, m, 16-H), 1.80 (1H, m, 12-H),
2.24 (1H, d, J=13.8 Hz, 19-H), 2.61 (1H, br-d, J=12.6 Hz, 12-H),
3.12 (1H, m, 22-H), 3.19 (1H, m, 3-H), 3.26 (1H, dd, J=7.8, 10.8 Hz, 24-H),
3.80 (1H, dd, J=2.4, 10.8 Hz, 24-H), 4.06 (1H, dd, J=2.4, 7.8 Hz, 24-OH)),
4.26 (1H, d, J=4.8 Hz, 22-OH), 4.95 (1H, d, J=4.2 Hz, 3-OH)
The sample was dissolved in deuterated dimethyl sulfoxide in ¹H-NMR, and the chemical shift of the residual proton of deuterated dimethyl sulfoxide was shown as 2.51 ppm. Figure 23 shows ¹H-NMR spectrum of the fraction **a** derived from the acid hydrolyzate of soybean embryo. In Figure 23, the axis of abscissas is the chemical shift, and the axis of ordinates is the intensity of signal.
¹³C-NMR : δ17.3 (25-C), 17.4 (28-C), 18.1 (26-C), 19.6 (6-C), 22.1 (27-C), 22.4 (11-C), 23.8 (23-C), 25.8, 33.1 (29 and 30-C), 26.1 (12-C), 26.6 (15-C), 28.2 (2-C), 32.7 (20-C), 34.0 (16-C), 35.8 (7-C), 37.5 (10-C), 38.6 (19-C), 39.3 (1-C), 40.9 (17-C), 41.5 (8-C), 43.1 (4-C), 44.7 (14-C), 44.8 (21-C), 51.0 (9-C), 56.3 (5-C), 63.8 (24-C), 76.6 (22-C), 79.5 (3-C), 133.0 (18-C), 136.9 (13-C)
The sample was dissolved in deuterated dimethyl sulfoxide in ¹³C-NMR, and the chemical shift of deuterated dimethyl sulfoxide was shown as 40.2 ppm. Figure 24 shows ¹³C-NMR spectrum of the fraction **a** derived from the acid hydrolyzate of soybean embryo. In Figure 24, the axis of abscissas is the chemical shift, and the axis of ordinates is the intensity of signal.
As described above, as a result of the analyses of the MS spectrum and NMR spectrum for the fraction a derived from the acid hydrolyzate of soybean embryo, it was identified that the fraction **a** derived from the acid hydrolyzate of soybean embryo is 13(18)-oleanene-3,22,24-triol (molecular weight: 458). The compound is a compound n in Table 1 mentioned above.
(5) The enhancing activity for NGF production of the compound n was assayed in the same manner as in item (2) of Example 2. The amount added to a medium was set so as to have a final concentration as shown in Table 3. As a result, it was clarified that the compound n has enhancing activity for NGF production. The results are shown in Table 3. The amount of NGF production of the control was 0.372 ng/mL.

**Table 3**

| Compound of the Present Invention | Amount of NGF Production (%) | | |
|---|---|---|---|
| | Concentration of Sample (mM) | | |
| | 0 | 25 | 50 |
| Compound n | 100 | 197.9 | 522.6 |
| The amounts of NGF production in the sample-added group are expressed as % when the amounts of NGF production in the sample-non-added group (control) are regarded as 100%. | | | |

### Example 17 Enhancing Activity for NGF Production of Glycyrrhizin

The enhancing activity for NGF production of glycyrrhizin (manufactured by Wako Pure Chemical Industries) was assayed in the same manner as in item (2) of Example 2. The amount added to a medium was set so as to have a final concentration as shown in Table 4. As a result, it was clarified that glycyrrhizin has enhancing activity for NGF production. The results are shown in Table 4. The amount of NGF production of the control was 0.484 ng/mL.

**Table 4**

| Compound of the present invention | Amount of NGF Production (%) | | | |
|---|---|---|---|---|
| | Concentration of Sample (mM) | | | |
| | 0 | 0.25 | 0.5 | 1.0 |
| Glycyrrhizin | 100 | 113.0 | 204.3 | 261.0 |
| The amounts of NGF production in the sample-added group are expressed as % when the amounts of NGF production in the sample-non-added group (control) are regarded as 100%. | | | | |

### INDUSTRIAL APPLICABILITY

According to the present invention, there is provided a medicament, foodstuff or feed, containing in a high content a saponin compound, a sapogenin compound or a salt thereof which is derived from an edible plant, and is safe. These are useful in treatment, prevention or amelioration of symptoms of cranial nerve-derived diseases by their enhancing action for NGF production. Especially, the foodstuff can enable intake of a given amount of a saponin compound or the like on a daily basis, so that the foodstuff is very useful as a foodstuff for maintaining homeostasis of a living body or a foodstuff for enhancing NGF production.

## Claims

1. A compound represented by the following general formula (I): 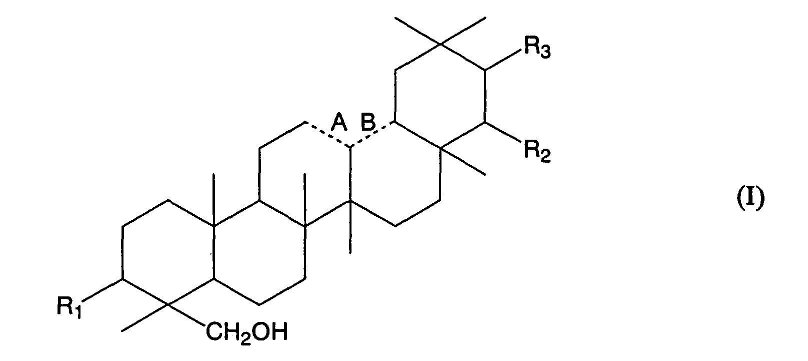 wherein of the bond lines indicated by dotted lines, when A is a double bond and B is a single bond, R₁ is -O-GlcUA-Gal-Glc, R₂ is -O-Ara-2-AcXyl, -O-Ara-3-AcXyl, -O-Ara-4-AcXyl, -O-Ara-2,3-diAcXyl, -O-Ara-2,4-diAcXyl, -O-Ara-3,4-diAcXyl or -O-Ara-3,4,6-triAcGlc, and R₃ is -OH; alternatively, when A is a single bond and B is a double bond, R₁ is -OH, R₂ is -OH and R₃ is -H, wherein GlcUA is glucuronic acid residue, Gal is galactose residue, Glc is glucose residue, Ara is arabinose residue, AcXyl is acetylated xylose residue, AcGlc is acetylated glucose residue,
or a salt thereof.

2. A therapeutic agent or prophylactic agent, **characterized in that** the therapeutic agent or prophylactic agent comprises as an effective ingredient the compound as defined in claim 1 and/or a pharmacologically acceptable salt thereof, wherein the agent is for a disease showing sensitivity to the above compound.

3. An enhancing agent for nerve growth factor production, **characterized in that** the enhancing agent comprises as an effective ingredient the compound as defined in claim 1 and/or a salt thereof.

4. A food, beverage or feed, **characterized in that** the food, beverage or feed comprises as an effective ingredient the compound as defined in claim 1 and/or a salt thereof.

5. The food, beverage or feed according to claim 4, wherein the food, beverage or feed is a food, beverage or feed used for enhancing nerve growth factor production.

6. A therapeutic agent or prophylactic agent for a disease requiring enhancement of nerve growth factor production upon treatment or prevention, **characterized in that** the therapeutic agent or prophylactic agent comprises as an effective ingredient a compound selected from the group consisting of soyasaponin compounds, soyasapogenin compounds, glycyrrhizin and pharmacologically acceptable salts thereof.

7. The therapeutic agent or prophylactic agent according to claim 6, wherein the soyasaponin compound and the soyasapogenin compound are a soyasaponin compound and a soyasapogenin compound which are represented by the following general formula (II): 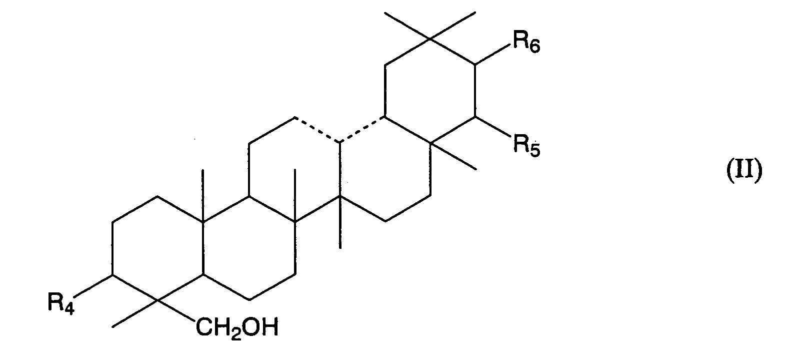 wherein the bond lines indicated by dotted lines are a single bond or a double bond; R₄ is -OH or -O-saccharide residue; R₅ is -OH, =O or -O-saccharide residue; and R₆ is -OH or -H.

8. An enhancing agent for nerve growth factor production, **characterized in that** the enhancing agent comprises as an effective ingredient a compound selected from the group consisting of soyasaponin compounds, soyasapogenin compounds, glycyrrhizin and salts thereof.

9. The enhancing agent according to claim 8, wherein the soyasaponin compound and the soyasapogenin compound are a soyasaponin compound and a soyasapogenin compound which are represented by the general formula (II) as defined in claim 7.

10. A food, beverage or feed for enhancing nerve growth factor production,
**characterized in that** the food, beverage or feed comprises as an effective ingredient a compound selected from the group consisting of soyasaponin compounds, soyasapogenin compounds, glycyrrhizin and salts thereof.

11. The food, beverage or feed according to claim 10, wherein the soyasaponin compound and the soyasapogenin compound are a soyasaponin compound and a soyasapogenin compound which are represented by the general formula (II) as defined in claim 7.
